(19) Europäisches Patentamt
European Patent Office
Office européen des brevets

(11) Publication number: **0 583 698 A2**

# (12) EUROPEAN PATENT APPLICATION

(21) Application number: **93112581.9**

(51) Int. Cl.5: **C07F 9/117**, C07F 9/32, A61K 31/66

(22) Date of filing: **05.08.93**

(30) Priority: **18.08.92 FR 9210108**

(43) Date of publication of application: **23.02.94 Bulletin 94/08**

(84) Designated Contracting States: **AT BE CH DE DK ES FR GB GR IE IT LI LU MC NL PT SE**

(71) Applicant: **Bayer Pharma**
**Rue Bellocier**
**F-89103 Sens(FR)**

(72) Inventor: **Bischoff, Erwin, Dr.**
**Pahlkestrasse 73**
**D-42115 Wuppertal(DE)**
Inventor: **Gao, Zahn, Dr., c/o Bayer AG, Beijing Liaison Off.**
**Unit 3009,**
**30/F Jing Guang Center**
**Hu Jia Lou, Chaoyang District(CN)**
Inventor: **Wohlfeil, Stefan, Dr.**
**Tucherweg 25**
**D-40724 Hilden(DE)**
Inventor: **Hecker, Gabriele, Dr.**
**Am Jagdhaus 47**
**D-42113 Wuppertal(DE)**
Inventor: **Cleophax, Jeannine**
**2 Résidence Marceau**
**F-91120 Palaiseau(FR)**
Inventor: **Dubreuil, Didier**
**15 Av. de la Résistance**
**F-91260 Juvisy/Orge(FR)**
Inventor: **Gero, Stéphane**
**2 Allée des Rouges Gorges**
**F-91940 Les Ulis(FR)**
Inventor: **Olesker, Alice**
**42 Allée du Bocage de Beaudreville**
**F-91190 Gif-sur-Yvette(FR)**
Inventor: **Verre-Sebrie, Catherine**
**15 Résidence Courdimanche**
**F-91940 Les Ulis(FR)**
Inventor: **Vieira de Almeida, Mauro**
**Rue Eponina Peixoto Ribeiro, 415**
**Granjaria**
**BR-36770-Cataguazes-M.G.(BR)**
Inventor: **Vass, Georges**
**14 Résidence du Château de Courcelles**
**F-91190 Gif-sur-Yvette(FR)**

(74) Representative: **Dänner, Klaus, Dr. et al**
**Bayer AG**
**Konzernverwaltung RP**
**Patente Konzern**
**D-51368 Leverkusen (DE)**

## (54) Deoxycyclitol derivatives, process for their preparation and their use in medicaments.

(57) Deoxycyclitol derivatives of general formula:

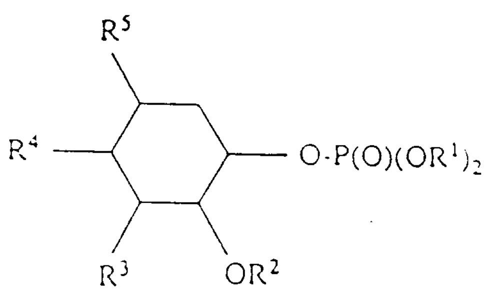

(I)

in which

EP 0 583 698 A2

- $R^1$ represents a hydrogen atom, a linear or branched alkyl group or a protecting group of the hydroxyl groups,
- $R^2$ represents hydrogen or a residue of formula $-P(O)(OR^6)_2$,
- $R^4$ represents a residue of formula $-OP(O)(OR^7)_2$ or A-O-,
- $R^3$ has the same meaning as $R^4$ or represents a hydrogen atom,
- $R^5$ represents a group of formula $-OP(O)(OR^8)_2$ or $-CH_2P(O)(OR^9)_2$,

and their salts, process for their preparation and their use in medicaments.

The present invention relates to new deoxycyclitol derivatives, to a process for their preparation and to their use in medicaments, in particular as anti-inflammatories.

It is known that D-myoinositol 1,4,5-triphosphate ($IP_3$) is the second messenger of a large number of receptors. The fastening of an effector molecule to the receptor leads to activation of phospholipase C which, from phosphatidylinositol 4,5-diphosphate, releases diacylglycerol (DAG) and D-myoinositol 1,4,5-triphosphate ($IP_3$). DAG is an activator of the protein kinase C, whereas $IP_3$ causes a momentary rise in the cytosolic $Ca^{2+}$ by fastening to an intracellular receptor [see Natural Product Reports (1990), 1-23; Phosphoinositides and Receptor Mechanisms, p. 1-24 (1986); Alan R. Liss, Inc. New York, Ed Putney, I.W. Fr., Agents and Actions, 19 (1986), 80-85; Br. J. Pharmac., 89 (1986), 803-807; TIBS 13 (1988), 148; TIBS (1989), 139].

The present invention relates to compounds of general formula (I):

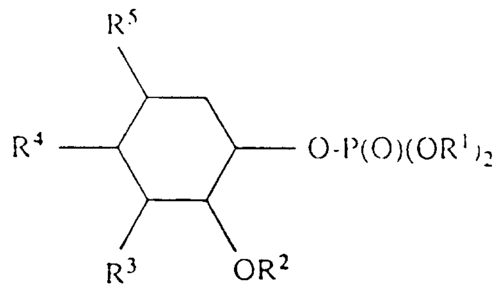

(I)

in which

| | |
|---|---|
| $R^1$ | represents a hydrogen atom, a linear or branched alkyl group having up to 8 carbon atoms or a protecting group of the hydroxyl groups, |
| $R^2$ | represents hydrogen or a residue of formula $-P(O)(OR^6)_2$, in which |
| $R^6$ | has the meaning shown above for $R^1$ and is identical to the latter or different from the latter, |
| $R^4$ | represents a residue of formula $-OP(O)(OR^7)_2$ or A-O-, in which |
| $R^7$ | also has the meaning shown above for $R^1$ and is identical to the latter or different from the latter, |
| A | represents a hydrogen atom or a linear or branched alkyl group having up to 10 carbon atoms or a linear or branched acyl group having up to 18 carbon atoms, |
| $R^3$ | has the meaning shown above for $R^4$ and is identical to the latter or different from the latter or represents a hydrogen atom, |
| $R^5$ | represents a group of formula $-OP(O)(OR^8)_2$ or $-CH_2P(O)(OR^9)_2$, in which |
| $R^8$ and $R^9$ | are identical or different and have the meaning shown above for $R^1$, |

and to their salts, with the condition that, when 2 or 3 of the substituents shown above represent the protecting group of the hydroxyl groups, the remaining substituents must not represent the residues of phosphoric acid $O-P(O)(OH)_2$ or $-P(O)(OH)_2$,

and

$R^1$ must not represent hydrogen when $R^2$ represents the group of formula $-P(O)(OH)_2$ and $R^3$, $R^4$ and $R^5$ each represent the group of formula $-OP(O)(OH)_2$.

As regards the salts, in the context of the present invention, salts are preferred which are faultless from the physiological view point. The salts of the compounds according to the invention which are faultless from the physiological view point may be salts or compounds according to the invention with inorganic acids, carboxylic acids or sulphonic acids. The salts with hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, propionic acid, lactic acid, tartaric acid, citric acid, fumaric acid, maleic acid or benzoic acid are particularly preferred.

There may also be mentioned the salts with the standard bases such as, for example, the alkali metal salts (for example the sodium or potassium salts), the alkaline-earth metal salts (for example the calcium or magnesium salts) or the ammonium salts derived from aqueous ammonia or from organic amines such as, for example, diethylamine, triethylamine, ethyldiisopropylamine, procaine, N-methylmorpholine or N-methylpiperidine, dihydroabietylamine or tris(hydroxymethyl)aminomethane (Tris).

The compounds according to the invention may exist in stereoisomeric forms which are symmetrical to each other in a mirror (enantiomers) or which are not symmetrical to each other in a mirror (diastereoisomers). The invention relates to the antipodes as well as the racemic forms and the mixtures of diastereoisomers. The racemic forms as well as the diastereoisomers may be separated in a known way into the homogeneous constituents from the stereoisomeric view point [see E.L. Eliel, Stereochemistry of Carbon Compounds, McGraw Hill, 1962].

Additionally, the substituents of the compounds according to the invention may occupy an axial or equatorial position on the cyclohexane skeleton. Preferably, the substituents occupy the positions which correspond to the stereochemistry of the known 1,4,5-$IP_3$ (see J. Chem. Soc., Chem. Commun., 1988, p. 1383-1385 and Pure Appl. Chem., 62 (10), p. 2031-2034, 1990).

In the context of the definition given above, the protecting group of the hydroxyl groups is generally a protecting group of the following series: trimethylsilyl, triethylsilyl, triisopropylsilyl, tert-butyldimethylsilyl, tert-butyldiphenylsilyl, triphenylsilyl, trimethylsilylethoxycarbonyl, benzyl, triphenylmethyl (Trityl), monomethoxytrityl (MMTr), dimethoxytrityl (DMTr), benzyloxycarbonyl, 2-nitrobenzyl, 4-nitrobenzyl, 2-nitrobenzyloxycarbonyl, 4-nitrobenzyloxycarbonyl, tert-butyloxycarbonyl, allyloxycarbonyl, 4-methoxybenzyl, 4-methoxybenzyloxycarbonyl, formyl, acetyl, trichloroacetyl, 2,2,2-trichloroethoxycarbonyl, 2,4-dimethoxybenzyl, 2,4-dimethoxybenzyloxycarbonyl, methoxymethyl, methylthiomethyl, methoxyethoxymethyl, [2-(trimethylsilyl)ethoxy]methyl, 2-(methylthiomethoxy)ethoxycarbonyl, tetrahydropyranyl, benzoyl, 4-methylbenzoyl, 4-nitrobenzoyl, 4-fluorobenzoyl, 4-chlorobenzoyl or 4-methoxybenzoyl.

The compounds of general formula (I) are preferred, in which

| | |
|---|---|
| $R^1$ | represents a hydrogen atom, a linear or branched alkyl group having up to 6 carbon atoms or a benzyl, benzyloxycarbonyl or 2- or 4-nitrobenzyl group, |
| $R^2$ | represents a hydrogen atom, an alkyl or benzyl group or a residue of formula $-P(O)(OR^6)_2$ in which |
| $R^6$ | has the meaning shown above for $R^1$ and is identical to the latter or is different therefrom, |
| $R^4$ | represents a residue of formula $-OP(O)(OR^7)_2$ or A-O-, in which |
| $R^7$ | also has the meaning given above for $R^1$ and is identical to the latter or is different therefrom, |
| A | represents a hydrogen atom, a benzyl group or a linear or branched alkyl group having up to 8 carbon atoms or a linear or branched acyl group having up to 16 carbon atoms, |
| $R^3$ | has the meaning shown above for $R^4$ and is identical to the latter or is different therefrom or represents a hydrogen atom, |
| $R^5$ | represents a group of formula $-OP(O)(OR^8)_2$ or $-CH_2P(O)(OR^9)_2$, in which |
| $R^8$ and $R^9$ | are identical or different and have the meaning shown above for $R^1$, |

and their salts, with the condition that, when 2 or 3 of the substituents shown above represent the protecting group of the hydroxyl groups, the remaining substituents must not represent the residues of phosphoric acid $O-P(O)(OH)_2$ or $-P(O)(OH)_2$,

and

$R^1$ must not represent a hydrogen atom when $R^2$ represents the group of formula $-P(O)(OH)_2$ and $R^3$, $R^4$ and $R^5$ each represent the group of formula $-OP(O)(OH)_2$.

The compounds of general formula (I) are particularly preferred

in which

| | |
|---|---|
| $R^1$ | represents a hydrogen atom, a linear or branched alkyl group having up to 5 carbon atoms or or a benzyl or alkyl group, |
| $R^2$ | represents a hydrogen atom, a benzyl group or a residue of formula $-P(O)(OR^6)_2$, in which |
| $R^6$ | has the meaning shown above for $R^1$ and is identical to the latter or is different therefrom, |
| $R^4$ | represents a residue of formula $-OP(O)(OR^7)_2$ or A-O-, in which |
| $R^7$ | also has the meaning shown above for $R^1$ and is identical to the latter or is different therefrom, and |
| A | represents a hydrogen atom, a benzyl group or a linear or branched alkyl group having up to 6 carbon atoms or a linear or branched acyl group having up to 14 carbon atoms, |
| $R^3$ | has the meaning shown above for $R^4$ and is identical to the latter or is different therefrom or represents a hydrogen atom, |
| $R^5$ | represents a group of formula $-OP(O)(OR^8)_2$ or $-CH_2P(O)(OR^9)_2$, in which |
| $R^8$ and $R^9$ | are identical or different and have the meaning shown above for $R^1$, |

and their salts, with the condition that, when 2 or 3 of the substituents shown above represent the protecting group of the hydroxyl groups, the remaining substituents must not represent the residues of phosphoric acid $O-P(O)(OH)_2$ or $-P(O)(OH)_2$,

and

$R^1$ must not represent a hydrogen atom when $R^2$ represents the group of formula $-P(O)(OH)_2$ and $R^3$, $R^4$ and $R^5$ each represent the group of formula $-OP(O)(OH)_2$.

The present invention also relates to processes for the preparation of the deoxycyclitol derivatives of general formula (I) according to the invention in which

[A] compounds of general formula (II)

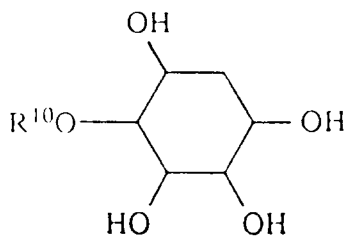

(II)

in which

$R^{10}$ represents one of the protecting groups of the hydroxyl groups mentioned above, preferably a benzyl group,

are reacted with compounds of general formula (III)

$((CH_3)_2CH)_2N\text{-}P(OR^{11})_2$ (III)

in which

$R^{11}$ has the meaning shown above for $R^{10}$ and is identical to the latter or is different therefrom,

in inert solvents, optionally in the presence of a base and then of an oxidising agent in order to form the compounds of general formula (IV)

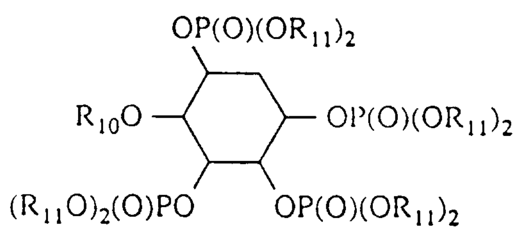

(IV)

in which

$R^{10}$ and $R^{11}$ have the meaning shown above,

or

compounds of general formula (V)

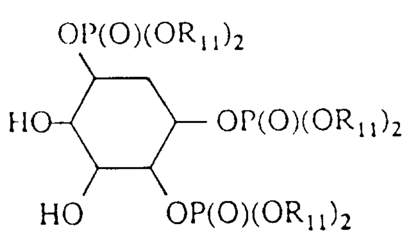

(V)

in which

$R^{11}$ has the meaning shown above,

are reacted with tetrabenzyl pyrophosphate, in inert solvents, in the presence of a base, in order to form the compounds of general formula (IVa)

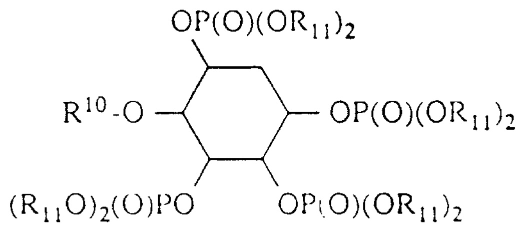

(IVa)

in which

$R^{10}$ and $R^{11}$ have the meaning shown above,

and then the protecting groups $R^{11}$ are removed by usual methods, preferably by catalytic hydrogenation, in the case of the compounds of general formula (IV) in the presence of tris(hydroxymethyl)-aminomethane,

or else

[B] when $R^3$ and/or $R^4$ represent the residue of formula A-O-, compounds of general formula (V) are first reacted with the compounds of general formula (VI)

A'-B (VI)

in which

A' has the meaning shown above for A, but does not represent a hydrogen atom, and

B represents a hydroxyl group or a typical leaving group such as tosylate, mesylate, chlorine, bromine or iodine, preferably chlorine or iodine,

in inert solvents, optionally in the presence of a base and/or of an additional reactant, in order to form the compounds of general formula (VII) or (VIIa)

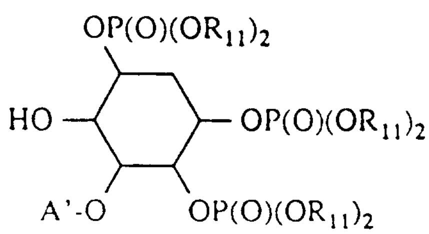

(VII)

or

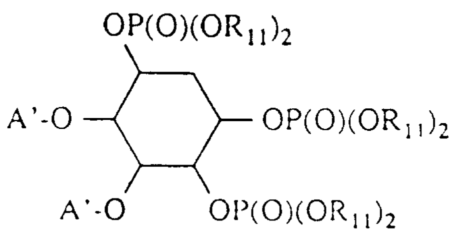

(VIIa)

in which

$R^{11}$ and A' have the meaning shown above,

or else

compounds of general formula (VIII)

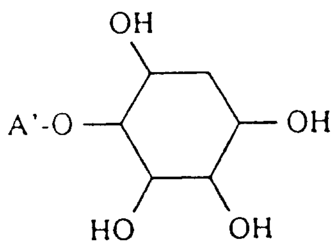

(VIII)

in which

A' has the meaning shown above,

are first of all converted to the compounds of general formula (IVb) by phosphorylation as described in [A],

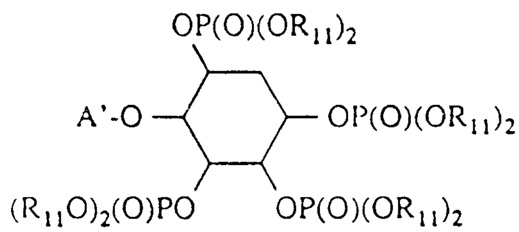

(IVb)

in which

A' and $R^{10}$ have the meaning shown above,

and, in a last stage, the various protecting groups of the compounds of general formulae (VII), (VIIa) and (IVb) are removed by the process described in [A], or else

[C] when $R^3$ represents a hydrogen atom,

compounds of general formula (IX)

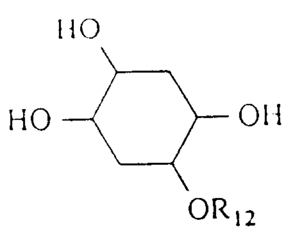

(IX)

in which

$R^{12}$ has the meaning shown above for $R^{10}$ and $R^{11}$ and is identical to these or is different therefrom,

are converted to the compounds of general formula (X) by phosphorylation according to the process described in [A]

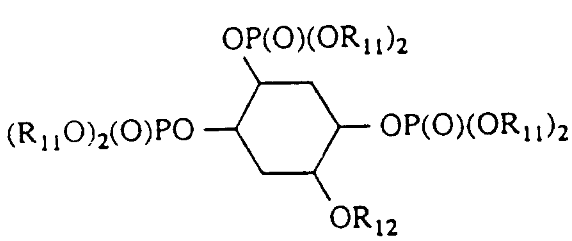

(X)

in which

$R^{11}$ and $R^{12}$ have the meaning shown above,

and then the protecting groups are removed by the process [A]

and

[D] when $R^5$ represents the residue of formula $-CH_2P(O)(OR^9)_2$, compounds of general formula (XI)

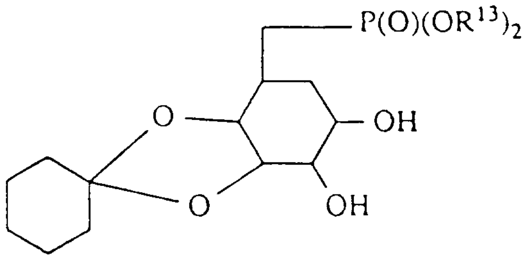

(XI)

in which

$R^{13}$ represents a $C_1$-$C_4$ alkyl group

are first reacted with the compounds of general formula (III) in order to form the compounds of general formula (XII)

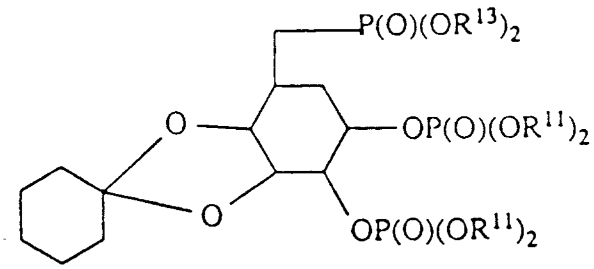

(XII)

in which

$R^{11}$ and $R^{13}$ have the meaning shown above,

and, in a later stage, the hydroxyl functional groups are freed with sodium in liquid ammonia with removal of all the protecting groups ($R^{11}/R^{13}$) or by catalytic hydrogenation with the residues $R^{13}$ being retained.

The processes according to the invention may be illustrated by the following scheme:

[A]

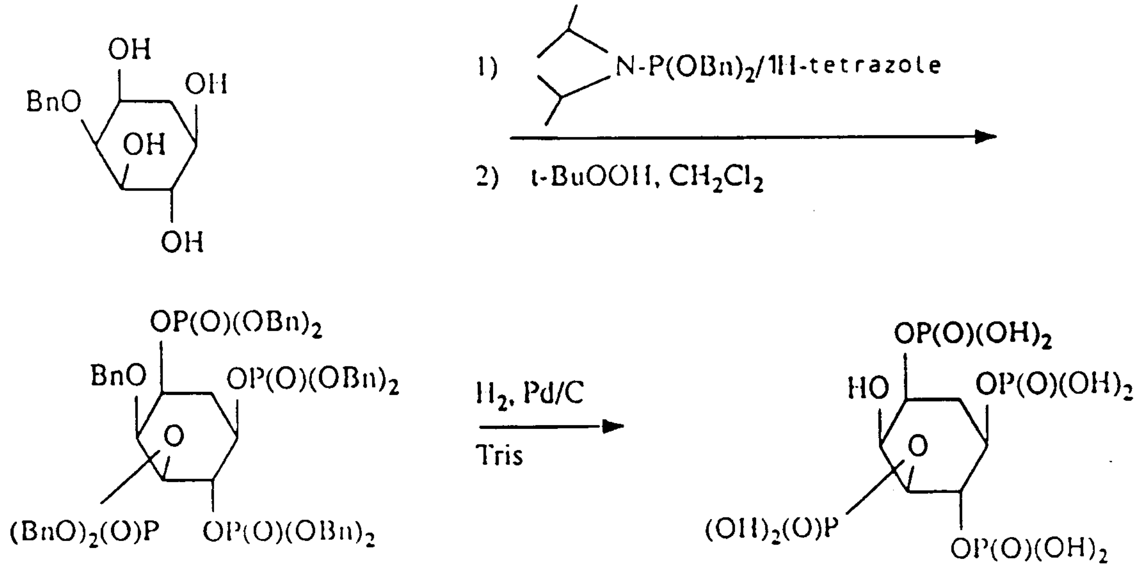
1)
N-P(OBn)2/1H-tetrazole
2) t-BuOOH, CH2Cl2
H2, Pd/C
Tris

[A]

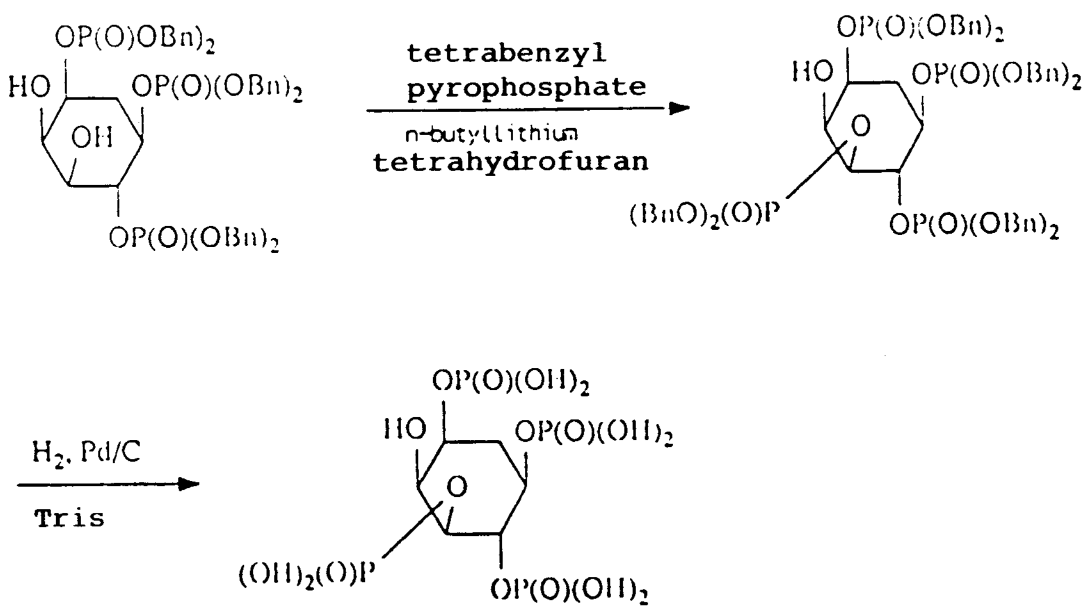
tetrabenzyl
pyrophosphate
n-butyllithium
tetrahydrofuran
H2, Pd/C
Tris

[B]

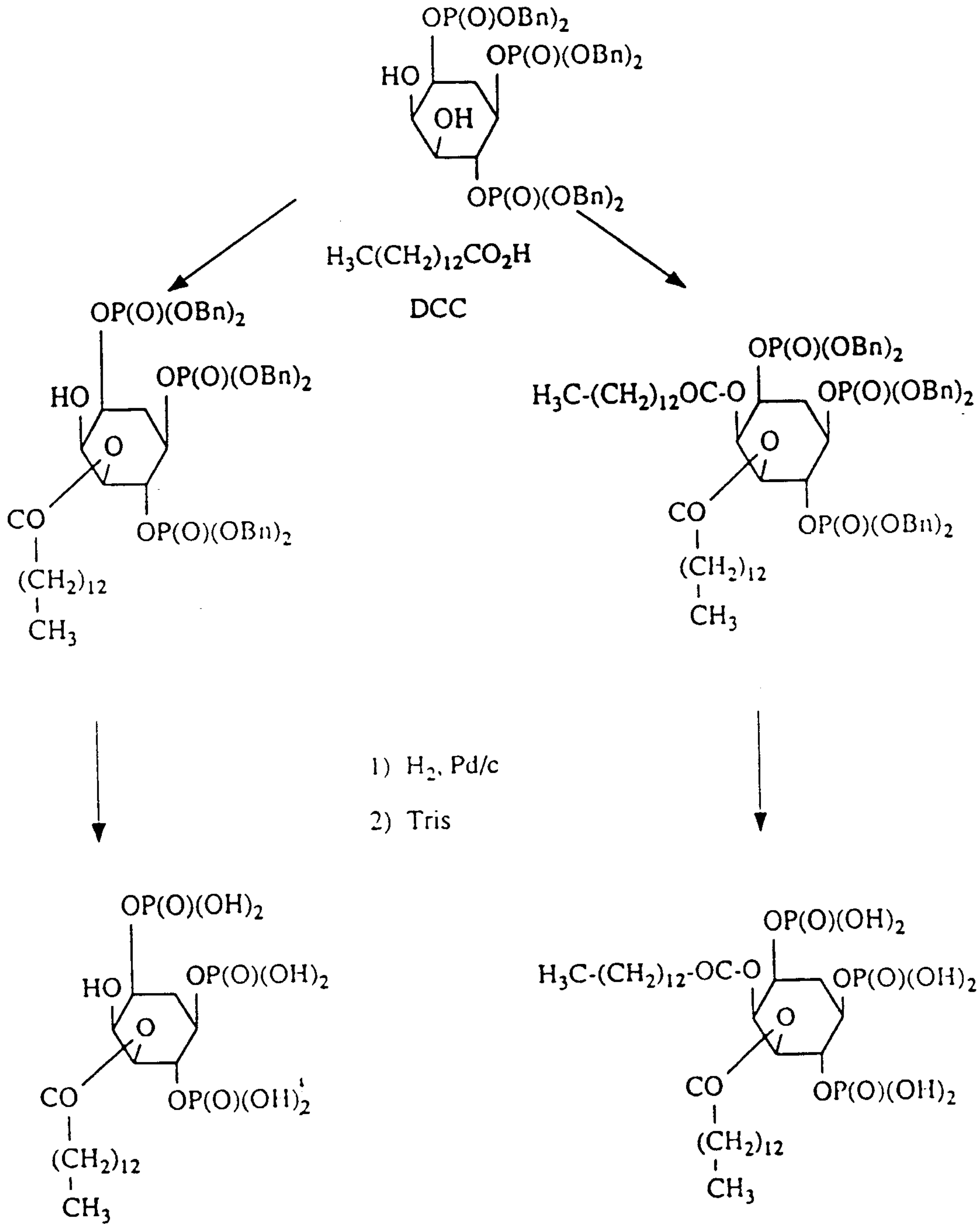

[C]

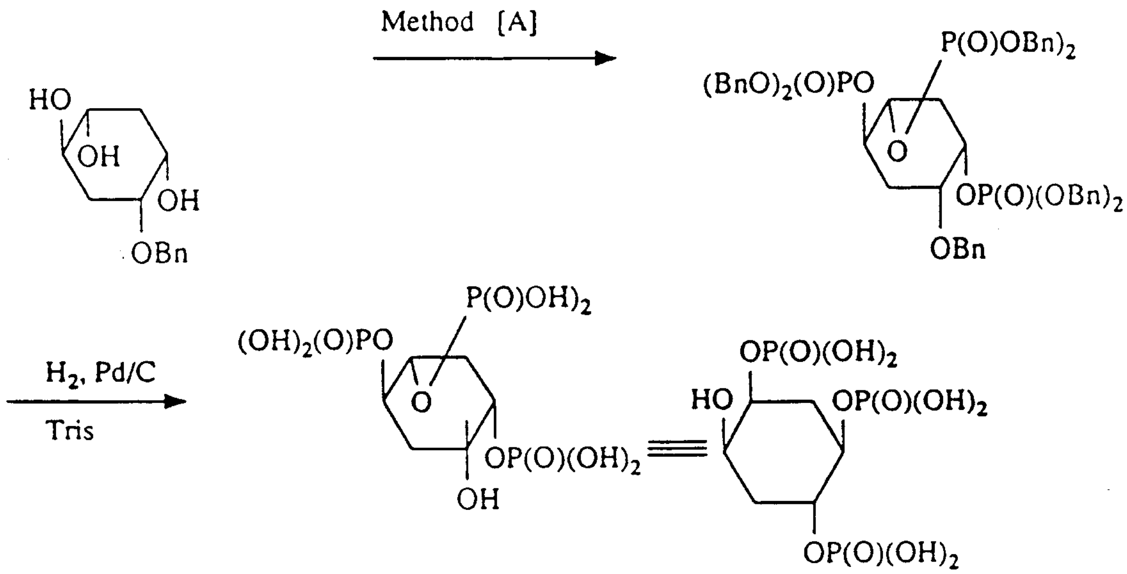


[D]

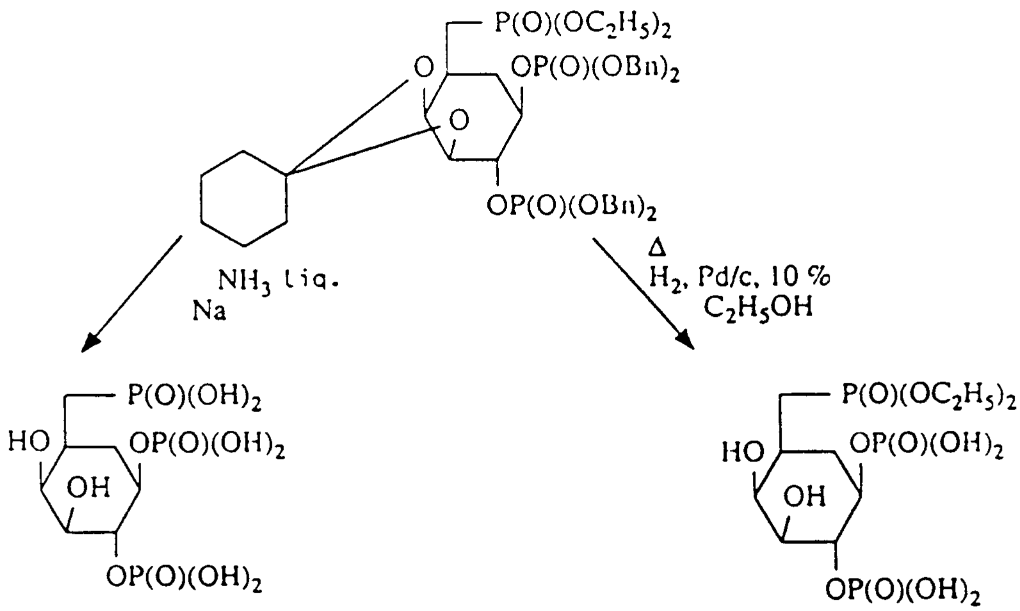


Bn = benzyl

Tris = tris(hydroxymethyl)aminomethane

The inert organic solvents which do not undergo change under the conditions of the reaction may be used as solvents for Processes [A] to [D]. The halogenated hydrocarbons such as dichloromethane, trichloromethane, tetrachloromethane, 1,2-dichloroethane, trichloroethane, tetrachloroethane or trichloroethylene, the hydrocarbons such as benzene, xylene, toluene, hexane, cyclohexane or petroleum fractions, nitromethane, dimethylformamide, acetonitrile or phosphoric acid hexamethyltriamide belong to these solvents. It is also possible to use mixtures of these solvents. Dichloromethane and acetonitrile are particularly preferred.

The standard basic compounds are suitable as bases for Processes [A] to [D]. They are preferably the alkali metal and alkaline-earth metal hydroxides such as lithium hydroxide, sodium hydroxide, potassium

hydroxide or barium hydroxide, the alkali metal hydrides such as sodium hydride, the alkali metal or alkaline-earth metal carbonates such as sodium carbonate or potassium carbonate, the alkali metal alkoxides such as sodium methoxide or ethoxide, potassium methoxide or ethoxide or potassium tert-butoxide, or the organic amines such as benzyltrimethylammonium hydroxide, tetrabutylammonium hydroxide, pyridine, triethylamine, N-methylpiperidine, 1H-tetrazole or tris(hydroxymethyl)aminomethane. 1H-Tetrazole and tris(hydroxymethyl)aminomethane are preferred.

The base is used in a quantity of 1 to 3 mol, preferably of 1 to 1.5 mol, per mole of OH group of the compound of general formula (II) and (IV).

The oxidising agents which are suitable are the oxidising agents such as tert-butyl hydroperoxide, metachloroperbenzoic acid or iodopyridine. tert-Butyl hydroperoxide is preferred.

Generally, Processes [A] to [D] are used in a temperature range from 0 to 150 °C, preferably from 25 to 40 °C.

Generally, Processes [A] to [D] are used at normal pressure. However, it is possible to use them at reduced pressure or under pressure (for example in a pressure range from $0.5 \times 10^5$ Pa to $5 \times 10^5$ Pa (0.5 to 5 bar).

Hydrogenolysis is carried out in order to remove the protecting groups, in particular the protecting groups of benzyl type, using hydrogen in inert solvents, such as the alcohols, ethers or halogenated hydrocarbons or their mixtures, with catalysts such as Raney nickel, palladium, palladium-on-animal-charcoal or platinum. Palladium-on-animal-charcoal is preferred [see Th. Greene: "Protective Groups in Organic Synthesis", J. Wiley & Sons, 1981, New York].

The catalyst is used in a quantity of 0.5 to 1 mol, preferably of 0.1 to 0.2 mol per mole of compound of general formulae (IV), (IVa), (IVb), (VIII), (IX) or (X).

Hydrogenolysis is generally carried out in a temperature range from 0 to 150 °C, preferably from 25 to 40 °C.

Hydrogenolysis generally takes place at normal pressure. However, it is also possible to use the process at reduced pressure or under pressure (for example in a range from $0.5 \times 10^5$ Pa to $5 \times 10^5$ Pa (0.5 to 5 bar).

For the reaction with tetrabenzyl pyrophosphate [A], the ethers mentioned above may be used, such as, for example, tetrahydrofuran, dioxane or ethyl ether. Tetrahydrofuran is preferred.

The standard basic compounds for the basic reactions may be used as bases. They are preferably alkali metal or alkaline-earth metal hydroxides such as, for example, sodium hydroxide, potassium hydroxide or barium hydroxide, alkali metal carbonates such as sodium carbonate, sodium hydrogencarbonate or potassium carbonate, or alkali metal alkoxides such as sodium methoxide, sodium ethoxide, potassium methoxide, potassium ethoxide or potassium tert-butoxide, or amides such as sodamide or lithium diisopropylamide, or else organolithium compounds such as phenyllithium or butyllithium. n-Butyllithium is preferred.

The base is used in a quantity of 1 to 3 mol, preferably of 1 to 1.5 mol, per mole of compound of general formula (VI).

The compounds of general formula (II), which are in part known and in part novel, may be prepared in a way analogous to the known processes [see Tetrahedron, vol. 46, No. 13, 14, p. 4995-5020, 1990].

The compounds of general formula (III) are known per se or may be prepared by the usual processes [see Helv. Chimica Acta, 1987, 70, 175-186].

The compounds corresponding to the general formulae (IV), (IVa), (IVb) and (VII), (VIIa), (X) and (XII) are novel and may be prepared by the processes mentioned above.

The compounds of general formula (VI) are known [see Beilstein, 2, 173; 2, 174].

The compounds of general formula (VIII) are likewise known per se or may be prepared by the usual processes [see Tetrahedron, vol. 46, No. 13, 14, p. 4995-5026, 1990].

The compounds of general formula (V) are known per se (see TH, vol. 46, p. 4995-5026, 1990], but they may be prepared according to a new process, in which compounds of general formula (XIII)

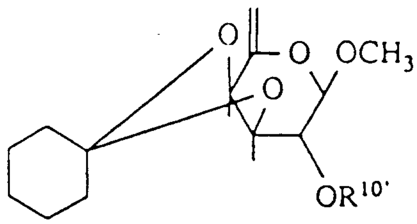

(XIII)

in which

$R^{10'}$ has the meaning shown above for $R^{10}$,

are first of all reacted with mercuric chloride and thiourea, in the presence of acetone and water, in order to form the compounds of general formula (XIV)

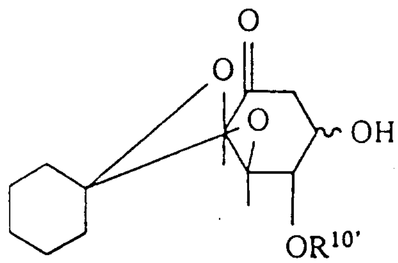

(XIV)

in which

$R^{10'}$ has the meaning shown above,

in a later stage, the carbonyl functional group is selectively reduced to a hydroxyl functional group with cerous chloride and sodium borohydride in methanol or with lithium borohydride in tetrahydrofuran, the hydroxyl functional group occupying an axial or equatorial position depending on the reducing agent and the stereo-chemistry of the other substituents on the ring, then, in a Mitsunobu reaction, the two free hydroxyl functional groups are blocked and take the same steric configuration, and, in a final stage, a phosphorylation by the method shown in Process [A] takes place and the protecting groups are removed.

The process according to the invention may be illustrated by the following scheme:

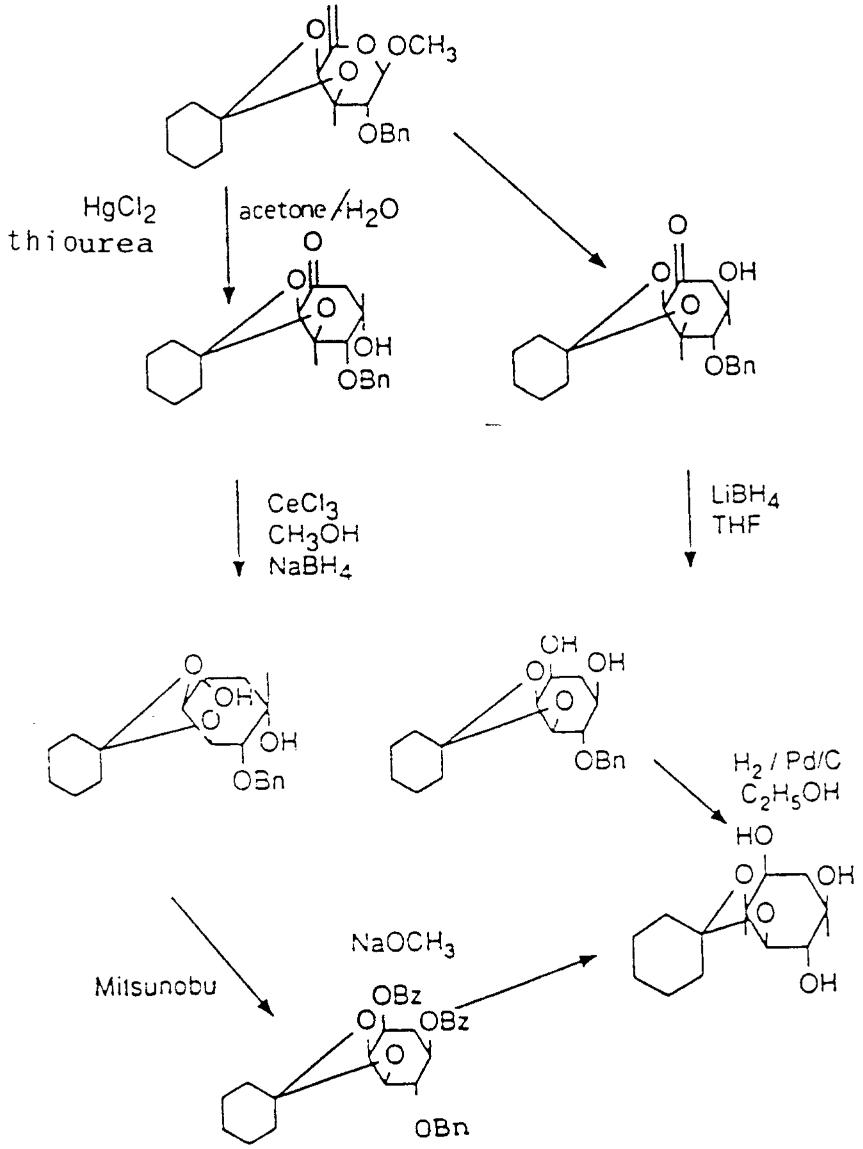
HgCl2
thiourea
acetone/H2O
CeCl3
CH3OH
NaBH4
LiBH4
THF
H2 / Pd/C
C2H5OH
NaOCH3
Mitsunobu

Bz = 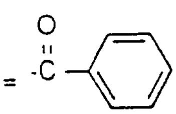

Bn = 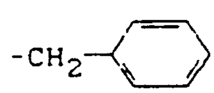

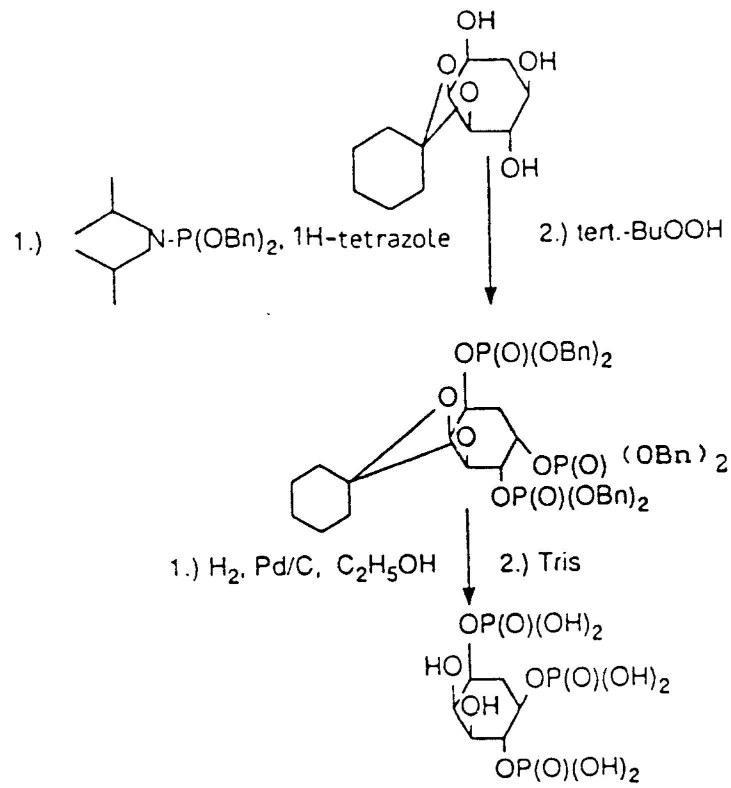


In general, the process is used in a temperature range between -10 °C and the boiling temperature of the solvent, preferably acetone, tetrahydrofuran and methanol, and preferably in a range from 0 to +100 °C.

In general, the process is carried out at normal pressure. However, it is possible to use the process at reduced pressure or under pressure (for example from $0.3 \times 10^5$ Pa to $3 \times 10^5$ Pa (0.3 to 3 bar)).

The compounds of general formula (XIII) are novel and may be prepared, for example, by reacting the corresponding methylbrominated compound with sodium hydride or caesium fluoride in dimethylformamide.

The compounds of general formula (XIV) are known per se and may be prepared in the way described above.

The compounds according to the invention which correspond to the general formula (I) exhibit a broad spectrum of pharmacological action which was unforeseeable.

The compounds according to the invention are phospholipase C inhibitors which additionally influence the metabolism of $IP_3$. They competitively displace $IP_3$ from the $IP_3$ receptor. The adrenergic receptors and the angiotensin II receptor are coupled to $IP_3$ as a second messenger. The release of $IP_3$ also exerts an influence on the blood platelet aggregation which is induced by thrombin.

The compounds according to the invention or their salts and isomers exhibit a pharmacological action spectrum which is advantageous and unforeseeable and they may be used as active principles in medicaments. They exhibit an anti-aggregation effect on thrombocytes. They may be used for the treatment of thrombo-embolic diseases and ischaemias, of transient and ischaemic attacks, and of peripheral disorders of the blood circulation.

Moreover, they exert an influence on the tonus of the smooth muscles. They may thus also be used in medicaments with a view to influencing pathologically modified blood pressure, as coronary therapeutic agents and for the treatment of cardiac insufficiency.

Thus, the compounds according to the invention are suitable for the treatment and the prevention of diseases of the respiratory tract, such as allergies, asthma or bronchitis, of inflammations, of rheumatisms, of arteriosclerosis and of dermatoses, such as psoriasis and inflammatory dermatoses. They are suitable in particular for the treatment and the prevention of all the diseases which are accompanied by inflammatory processes.

The pharmacological effects of the substances according to the invention are determined by the following methods:

1) Competition studies with radiolabelled 1,4,5-$IP_3$ on membrane preparations arising from dog cerebellum. BBRC 146 (1987), 1071-1078 [A.J. Willcocks, A.M. Cooke, B.V.L. Potter and S.R. Nahorski, William

W.Y. Lo and John Hughes, Neuroscience Letters, 81 (1987), 331-334].

2) Studies of the inhibition of the dephosphorylation of 1,4,5-$IP_3$ [A.M. Cooke, S.R. Nahorski and B.L.V. Potter, FEBS-Letters, 242 (1989), 373-377].

The pharmaceutical preparations which contain inert, nontoxic and pharmaceutically suitable adjuvants and vehicles as well as one or a number of compounds of general formula (I), or which consist of one or a number of active principles of formula (I), as well as the processes for producing these preparations, also form part of the present invention.

The active principles of formula (I) must be present in these preparations in a concentration of from 0.1 to 99.5 % by weight, preferably from 0.5 to 95 % by weight, of the total mixture.

Besides the active principles of formula (I), the pharmaceutical preparations may contain other pharmaceutical active principles.

The pharmaceutical preparations mentioned above may be obtained in the usual way by known methods, and contain, for example, one or more adjuvant(s) or vehicle(s).

In general, it has appeared advantageous to administer the active principle(s) of formula (I) in total quantities from approximately 0.01 to approximately 100 mg/kg, preferably in total quantities from approximately 1 mg/kg to 50 mg/kg, of body weight per 24 h, optionally in the form of several separate administrations, in order to obtain the desired result.

However, it may optionally be advantageous to depart from the quantities shown depending on the type and the body weight of the patient to be treated, on the individual behaviour with respect to medicaments, on the type and the seriousness of the disease, on the type of preparation and application, and on the time at which administration takes place and on the intervals separating the administrations.

GENERAL INSTRUCTIONS FOR THE PHOSPHORYLATION AND RELEASE PROCESS

I. Process A: Phosphoramidite process

Preparation of the phosphorylation agent dibenzyloxy(diisopropylamino)phosphine

$(C_6H_5CH_2O)_2P — N[CH(CH_3)_2]_2$

4.4 ml of phosphorus trichloride are added under argon to 30 ml of dry, freshly distilled ether. 14 ml of diisopropylamine in 30 ml of dry ether are added dropwise to the solution which has been cooled to -10 °C. The mixture is stirred for 1 h at -10 °C. It is then heated to 20 °C and filtered. At the end of 3 h, the filtrate is evaporated to dryness and distilled. 6.3 g of dichloro(diisopropylamino)phosphine are obtained. This intermediate product is dissolved under argon in 60 ml of dry acetonitrile. The solution is cooled to -10 °C and 13.7 ml of diisopropylethylamine are added. To this solution, which is left under argon at -10 °C, 6.6 ml of distilled benzyl alcohol dissolved in 40 ml of acetonitrile are added dropwise. The solution is stirred for 1.5 h. The temperature is brought to 20 °C. At the end of 12 h, the mixture is taken up in 150 ml of dichloromethane after evaporation to dryness. The organic phase is washed, first with a solution of sodium bicarbonate in water and then with water. After drying over $MgSO_4$ and evaporating to dryness, a yellow oil is obtained. 8.8 g of crude substance are thus obtained which substance consists essentially of the title compound.

1. Phosphorylation

a) A mixture A of x mg of alcohol to be phosphorylated and 2 equivalents (per hydroxyl group to be phosphorylated) of dibenzyloxy(diisopropylamino)phosphine and dried under vacuum for 1/2 h under 6.67 Pa (0.05 mm Hg).

b) 2 equivalents (with respect to the phosphinylation reactant) of tetrazole which, beforehand, has been sublimed and dissolved in the minimum amount of anhydrous acetonitrile are added under argon to Mixture A.

c) The solution is stirred under argon for 1 h at room temperature.

2. Oxidation (phosphorus III ----> phosphorus V)

The mixture is then diluted by addition of dichloromethane. 2 equivalents (with respect to the phosphinylation reactant) of tert-butyl hydroperoxide (t-BuOOH) are then added. The solution is stirred under argon for 3 h at room temperature.

3. Separation of the phosphorylation products

An aqueous solution of sodium thiosulphate and of sodium bicarbonate is added in order to neutralise the reaction. After extracting with dichloromethane and evaporating the organic phase to dryness, separation takes place on a silica gel plate or by RP 8 or RP 18 reverse phase.

II. Process B: Pyrophosphate process

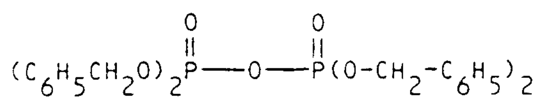

Preparation of the phosphorylation reactant: tetrabenzyl pyrophosphate

556 mg (2 mmol) of dibenzyl phosphate dissolved in a mixture of 2 ml of acetonitrile and 2 ml of anhydrous ether are added to 227 mg (1.1 mmol) of dicyclohexylcarbodiimide (DCC) dissolved in 1 ml of anhydrous ether. Acetonitrile is added until the mixture has completely dissolved. The mixture is stirred; at the end of 15 min, a white precipitate is formed. After filtering the precipitate and washing with dichloromethane, the filtrate is evaporated to dryness and recrystallised from hexane (overnight at room temperature). 250 mg of solid tetrabenzyl pyrophosphate appears. M.p.: 61-62 °C.

General phosphorylation process

x mg of alcohol are dissolved in the minimum amount of anhydrous tetrahydrofuran. The solution is left to cool to 0 °C, 1.1 equivalents of n-butyllithium (n-BuLi) are added per free hydroxyl group and the mixture is stirred for 5 min. 1.3 equivalents of tetrabenzyl pyrophosphate per hydroxyl group to be phosphorylated are then added at -40 °C. The solution is stirred for 1 h under argon. The mixture is then filtered on diatomaceous earth and eluted with ethyl acetate before being evaporated to dryness. The phosphorylated substances are isolated by separating on silica gel plates or on a Florisil column or an RP 8 column.

GENERAL INSTRUCTIONS FOR RELEASING THE DERIVATIVES OF 6-DEOXYCYCLITOL PHOSPHATE

1) Simultaneous release of the benzyl ester groups of the phosphates, and the benzyl ether and cyclohexylidene acetal groups of the cyclitols.

x mg of the derivative X of 6-deoxymyoinositol dibenzylphosphate, dissolved in the minimum amount of ethanol and 1 ml of distilled water, are hydrogenated for 1 h at a pressure of $34.5 \times 10^3$ Pa (5 psi) in the presence of an equivalent quantity of palladium-on-charcoal (10%). After filtering the catalyst on Whatman paper and eluting with distilled water, the organic solvent is evaporated. Part of the water is then removed. 2 equivalents of tris(hydroxymethyl)aminomethane (Tris) per phosphate to be neutralised are then added. The mixture is then lyophilised.

2) Release of the benzyl ester protecting groups on the phosphates and of the benzyl ether protecting groups on the cyclitols without hydrolysis of the cyclohexylidene.

x mg of the derivative of 6-deoxymyoinositol dibenzyl phosphate, dissolved in the minimum amount of anhydrous ethanol, are hydrogenated for 1 h at a pressure of $13.8 \times 10^3$ Pa (2 psi) in the presence of an equivalent quantity of palladium-on-charcoal (10%). The catalyst is removed by filtering on Whatman paper and eluting with water. 2 equivalents of Tris per phosphate are added and the aqueous solution is evaporated. The mixture is then lyophilised. The analogous phosphates are obtained in the form of Tris salts. Their purity is monitored on a cellulose plate (mobile phase: isopropanol/aqueous ammonia/$H_2O$: 5/1/4). Under these conditions, the Tris salt is replaced by aqueous ammonia and the phosphates are revealed in the form of ammonium salts.

Starting compounds

Example 1

3,4-O-cyclohexylidene-β-D-methylgalactopyranoside

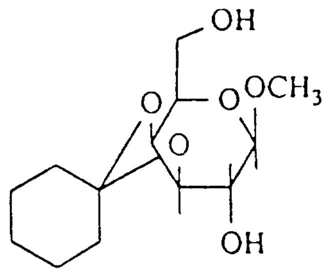

10 g (51 mmol) of β-D-methylgalactopyranoside are dissolved in 40 ml of dimethylformamide. 2 equivalents of dimethoxycyclohexane (13 ml) and 0.7 ml of sulphuric acid are added and the solution is stirred for 12 h. After neutralising with sodium bicarbonate and then filtering on diatomaceous earth (elution with ethyl acetate), the title compound is obtained with a yield of 90 % which crystallises from hexane.
M.p.: 120-121 °C.
$[\alpha]_D$ = +82° (0.98, $CH_3OH$)

Example 2

6-bromo-3,4-O-cyclohexylidene-6-deoxy-β-D-methylgalactopyranoside

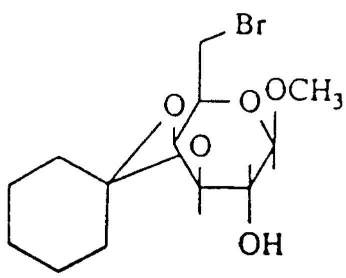

18 g (70 mmol) of triphenylphosphine and 18 g (55 mmol) of tetrabromomethane are added at 40 °C to a solution of 13.7 g (50 mmol) of the compound of Example 1 in 130 ml of anhydrous pyridine. The solution is then heated for 6 h at 60 °C. 20 ml of methanol are then added to the solution which has been cooled beforehand to room temperature. The pyridine is removed by codistillation with toluene (3 × 50 ml). The residue is taken up in ethyl acetate and evaporated several times in the presence of ether. The title compound is isolated by flash chromatography on silica gel: 13.5 g of compound are crystallised from an ethyl acetate/hexane mixture. The yield is 80 %.
M.p.: 122-123 °C.
$[\alpha]_D$ = +21° (1.01, $CH_3OH$).

Example 3

2-O-benzyl-6-bromo-3,4-O-cyclohexylidene-6-deoxy-β-D-methylgalactopyranoside

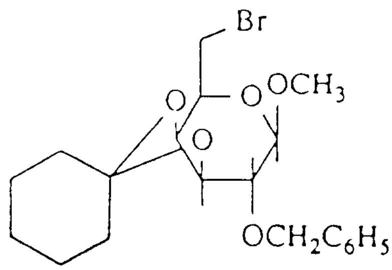

10 mg (166 mmol) of powdered potassium hydroxide (KOH) and 1 g of benzyltriethylammonium chloride (TEBAC) are added to 13.5 g (40 mmol) of the compound of Example 2, which is dissolved in 200 ml of methylene chloride. At the end of 10 min, 10 ml of benzyl bromide are added and the mixture is stirred vigorously for 12 h. 10 ml of methanol are then added to the mixture which is stirred for a further 1 h. The salts formed are removed by filtering on diatomaceous earth and the filtrate is evaporated to dryness. 13.7 g of the title compound are isolated by, flash chromatography on silica gel and are crystallised from hexane (yield: 90 %). M.p.: 94-95 °C. $[\alpha]_D$ - +46 ° (1, $CHCl_3$).

Example 4

2-O-benzyl-3,4-O-cyclohexylidene-6-deoxy-β-L-arabino-hex-5-enomethylpyranoside

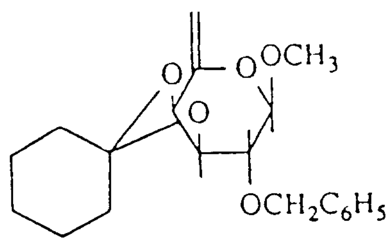

I. 1.12 g (46.8 mmol) of sodium hydride, in three portions, are added under argon to a solution of 10 g (23.4 mmol) of the compound of Example 3 in 20 ml of anhydrous dimethylformamide. The solution is stirred for 3 h and heated to 100 °C. The mixture is then cooled and 10 ml of methanol are added. Stirring is continued for a further hour and then extraction with dichloromethane takes place. The organic phase is evaporated to dryness. The title compound is isolated by flash chromatography on silica gel (ethyl acetate/hexane: 1/9). In this way, 7.5 g of product are isolated which product is crystallised from n-pentane (yield: 90 %).
M.p.: 61 °C.
$[\alpha]_D$ = -55 ° (1, $CHCl_3$).
II. 4.6 g (10.8 mmol) of the compound of Example 3 are dissolved under argon in 5 ml of anhydrous dimethylformamide. 3.3 g (21.6 mmol) of caesium fluoride (CsF), which was dried beforehand using a vane pump, are added with 492 mg (2.16 mmol) of benzyltriethylammonium chloride (TEBAC). The solution is stirred for 4 h at 120 °C. The mixture is then cooled to room temperature and extracted with dichloromethane. The organic phase is evaporated to dryness and then 3.2 g of the title compound are isolated by crystallising from n-pentane (yield: 85 %).
M.p.: 61-62 °C.
$[\alpha]_D$ = -55 ° (1, $CHCl_3$).

Examples 5 and 6

2-D-(2,3/4,5)-4-O-benzyl-2,3-O-cyclohexylidene-1-one-2,3,4,5-cyclohexanetetrol

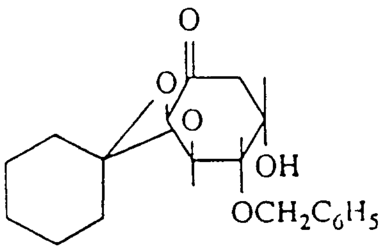

(Example 5)

2-D-(2,3,5/4)-4-O-benzyl-2,3-O-cyclohexylidene-1-one-2,3,4,5-cyclohexanetetrol

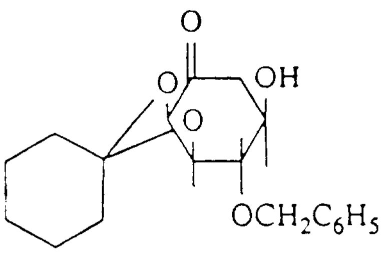

(Example 6)

1.7 equivalents (2.45 mmol, 665 mg) of mercuric chloride ($HgCl_2$) are added to 500 mg of the compound of Example 4 (1.44 mmol), which is dissolved in 18 ml of an acetone/water (2/1) mixture. The solution is stirred for 20 min and then 4 equivalents of thiourea (745 mg) with respect to the mercuric chloride are added. Stirring is continued for 2 h. After filtering on diatomaceous earth, washing the precipitate with acetone and evaporating the organic solvent, the residue is extracted with ethyl acetate. The organic phase is evaporated to dryness and 400 mg of a mixture of the two ketones 5 and 6 are obtained. The total yield of the reaction is 95 %. By separation by flash chromatography on silica gel (elution: ethyl acetate/hexane: 2/8), it is possible to isolate the two 6-deoxyinososes 5 and 6. The total yield after separation is greater than 85 %. The ratio between the 5 and 6 isomers is 1/1. Isomer 5 is eluted first and is in the form of a syrup. Isomer 6 is crystallised from n-pentane.

M.p.: 79-80 °C

$[\alpha]_D$ = -6 ° (1, $CHCl_3$) (Example 6)

$[\alpha]_D$ = +10 ° (1.03, $CHCl_3$) (Example 5).

Examples 7 and 8

4-O-benzyl-2,3-O-cyclohexylidene-6-deoxy-D-myoinositol

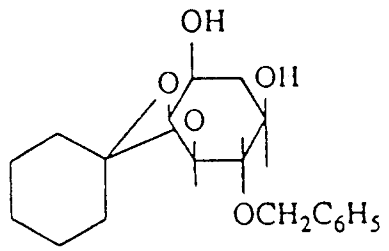

(Example 7)

3-O-benzyl-1,2-O-cyclohexylidene-5-deoxy-D-chiroinositol

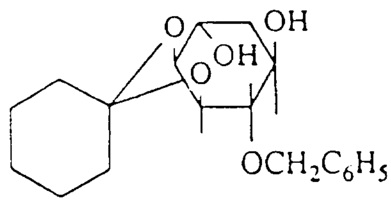

(Example 8)

1 g (3 mmol) of the compound of Example 6 is dissolved in 20 ml of anhydrous tetrahydrofuran. 142 mg of lithium borohydride ($LiBH_4$) are added to the solution which is stirred for 1 h at -78 °C under argon. 5 ml of a saturated aqueous sodium chloride solution are then added and the temperature is brought to 20 °C. Stirring is continued for 12 h at 20 °C. After evaporating to dryness, the residue is diluted a number of times with 30 ml of isopropanol and evaporated to dryness. The mixture is then taken up in ethyl acetate and filtered on diatomaceous earth. The filtrate is concentrated and the title compound of Example 7 is isolated by crystallising from an AcOEt/n-pentane mixture. 868 mg (2.6 mmol) are formed with a yield greater than 85 %.

M.p.: 124-125 °C.

$[\alpha]_D$ = -5 ° (0.9, $CHCl_3$).

The compound of Example 8 is obtained by isolation from the mother liquor with a yield of 10 %.

$[\alpha]_D$ = +20 ° (c = 1.1, $CHCl_3$).

Example 9

5,6-O-cyclohexylidene-3-deoxy-L-chiroinositol

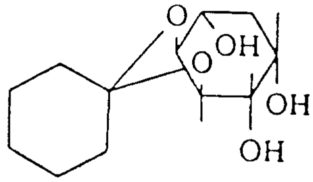

350 mg (1.05 mmol) of 1-O-benzyl-5,6-O-cyclohexylidene-3-deoxy-L-chiroinositol in 20 ml of ethyl acetate are hydrogenated for 1 h at a pressure of 20.7 × $10^3$ Pa (3 psi) in the presence of 350 mg of palladium-on-charcoal (10 %). The catalyst is removed by filtering on Whatman paper and eluting with ethanol. The filtrate is evaporated to dryness and the title compound is crystallised from chloroform. 250 mg (1.02 mmol) of product are obtained with a yield of 97 %.

M.p.: 136-137 °C.
$[\alpha]_D$ = -38 ° (c = 0.8, $CH_3OH$).

Example 10

2,3-O-cyclohexylidene-6-deoxy-D-myoinositol

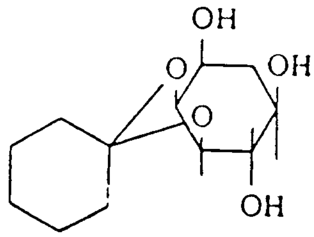

110 mg (0.33 mmol) of 4-O-benzyl-2,3-O-cyclohexylidene-6-deoxymyoinositol in a solution of 10 ml of ethyl acetate are hydrogenated for 2 h at a pressure of $34.5 \times 10^3$ Pa (5 psi) in the presence of 55 mg of palladium hydroxide on charcoal (20 %) and of 10 mg of calcium carbonate. The catalyst is removed by filtering on Whatman paper (elution with ethanol) and the filtrate is evaporated to dryness. 77 mg (0.31 mmol) of title compound are recrystallised from chloroform. (Yield: 95 %).
M.p.: 134-135 °C.
$[\alpha]_D$ = +42 ° (1.3, $CH_3OH$).

Example 11

5,6-O-cyclohexylidene-3-deoxy-L-chiroinositol 1,2,4-tris(dibenzyl phosphate)

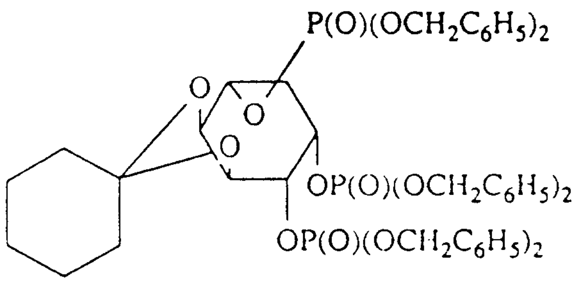

The title compound is prepared from the compound of Example 9 with a yield of 75 % by the general process set out above.
$[\alpha]_D$ = -14 ° (c = 0.63, $CHCl_3$).

Example 12

2,3-O-cyclohexylidene-6-deoxy-D-myoinositol 1,4,5-tris(dibenzyl phosphate)

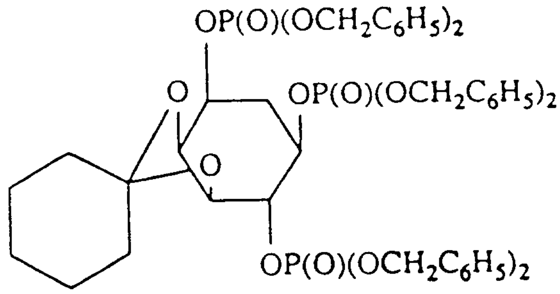

By Process A, the title compound is isolated from the compound of Example 10 with a yield greater than 75 %. The title compound crystallises from an AcOEt/n-pentane mixture.
M.p.: 76 ° C.
$[\alpha]_D$ = +4 ° (c = 0.8, $CHCl_3$).

Example 13

1,3,5-O-orthoformyl-6-deoxy-D-myoinositol

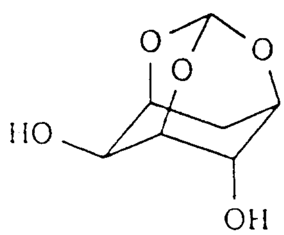

0.76 ml (4.65 mmol) of triethyl orthoformate and 86 mg of paratoluenesulphonic acid monohydrate are added under argon to a solution of 510 mg (3.1 mmol) of 6-deoxymyoinositol, which is dissolved in 10 ml of anhydrous dimethylformamide. The mixture is stirred for 12 h at 60 ° C. After neutralising with an aqueous sodium bicarbonate solution, the mixture is filtered on diatomaceous earth (elution: EtOH). The filtrate is evaporated to dryness. 500 mg (2.87 mmol) of title compound are isolated by crystallising from a MeOH/ethyl acetate/n-pentane mixture (yield: 93 %).
M.p.: 180-182 ° C.
$[\alpha]_D$ = +5 ° (0.48, $CH_3OH$).

Examples 14 and 15

2-O-benzyl-1,3,5-O-orthoformyl-6-deoxy-D-myoinositol

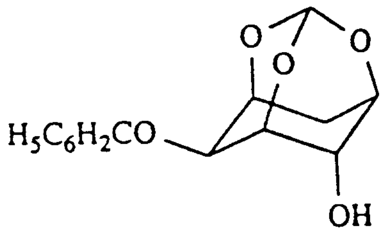

(Example 14)

2,4-di-O-benzyl-1,3,5-O-orthoformyl-6-deoxy-D-myoinositol

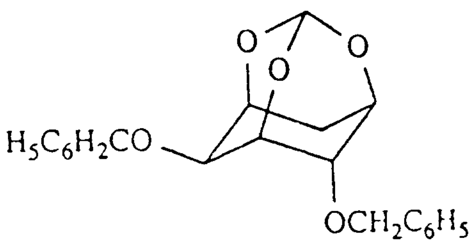

(Example 15)

452 mg (2.6 mmol) of compound of Example 13 are dissolved in 5 ml of anhydrous dimethylformamide. 81 mg (2.86 mmol) of sodium hydride (NaH) and 0.32 ml (2.86 mmol) of benzyl bromide are added to the solution which is stirred under argon for 1 h at 60 °C. After cooling to room temperature, 5 ml of methanol are added and stirring is continued for 1 h. The mixture is extracted with dichloromethane and the organic phase is evaporated to dryness. The compounds are not purified.

Examples 16 and 17

2-O-benzyl-6-deoxy-D-myoinositol

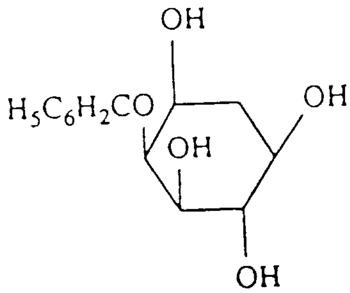

(Example 16)

2,4-di-O-benzyl-6-deoxy-D-myoinositol

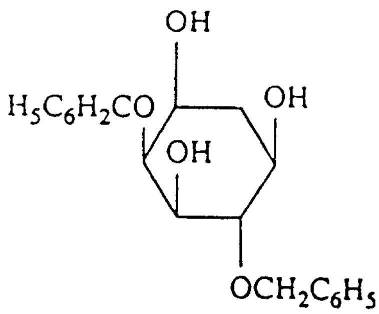

(Example 17)

0.5 g of the mixture of the compounds of Examples 14 and 15 is treated for 1 h at 60 °C with a 1N aqueous hydrochloric acid solution. The solution is evaporated to dryness. Purification of the title compounds is obtained by chromatography on Florisil (eluent: ethyl acetate/hexane: 8/2) in a ratio of 4 to 1 with a yield of 50 % (Example 16) and 22 % (Example 17).
Example 16 $[\alpha]_D$ = +5° (c = 0.98, $CH_3OH$);
Example 17 $[\alpha]_D$ = +3° (c = 1.1, $CH_3OH$).

Example 18

4-O-benzyl-6-deoxy-D-myoinositol

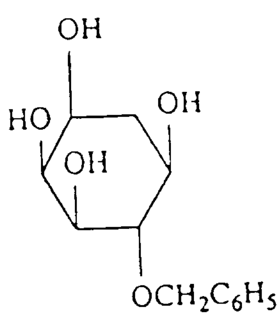

1 g (2.9 mmol) of 4-O-benzyl-2,3-O-cyclohexylidene-6-deoxymyoinositol is treated with a 1N aqueous hydrochloric acid solution. After evaporating to dryness, 780 mg of title compound are isolated by crystallising from an isopropanol/pentane mixture (yield: 95 %).

M.p.: 130-131 ° C.
$[\alpha]_D$ = +2° (c = 1, $CH_3OH$).

Example 19

4-O-benzyl-1,3,5-O-orthoformyl-6-deoxy-D-myoinositol

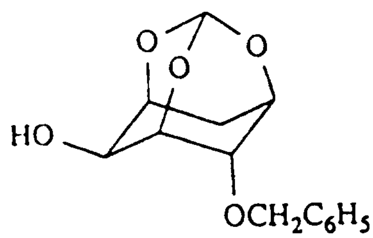

0.55 ml (3.3 mmol) of triethyl orthoformate and 76 mg of paratoluenesulphonic acid monohydrate are added to 470 mg (1.85 mmol) of compound of Example 18, which is dissolved in 5 ml of anhydrous dimethylformamide. The solution is stirred under nitrogen for 12 h at 60 ° C. It is then neutralised with an aqueous sodium bicarbonate solution. The mixture is filtered on diatomaceous earth and eluted with dichloromethane. The organic phase is evaporated to dryness. A yellow oil is formed which may be decoloured by treatment with charcoal in methanol. After stirring for 15 min, the mixture is filtered at 60 ° C on diatomaceous earth and evaporated to dryness (elution: $CH_2Cl_2$). Crystallisation from chloroform is then possible (12 h in a refrigerator), which makes it possible to isolate 476 mg (1.8 mmol) of title compound (yield: 97 %).
M.p.: 104-106 ° C.
$[\alpha]_D$ = +9° (c = 1.31, $CH_2Cl_2$).

Example 20

2-O-allyl-4-O-benzyl-1,3,5-O-orthoformyl-6-deoxy-D-myoinositol

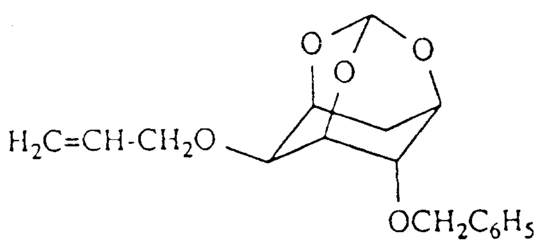

700 mg (2.65 mmol) of compound of Example 19 are dissolved in 5 ml of anhydrous dimethylformamide in a round-bottomed flask made of coloured glass. 95 mg (3.97 mmol) of sodium hydride (NaH) and 0.35 ml (3.9 mmol) of allyl bromide are added to the mixture. The solution is stirred for 1.5 h under argon at room temperature. 5 ml of methanol are then added and stirring is continued for 1 h. The mixture is extracted with dichloromethane and the organic phase is then evaporated to dryness. The residue is separated on a silica gel plate. 525 mg (1.73 mmol) of the title compound are thus obtained in the form of an oil (yield: 55 %).
$[\alpha]_D$ = +11° (c = 0.55, $CH_2Cl_2$).

Example 21

2-O-propyl-1,3,5-O-orthoformyl-6-deoxy-D-myoinositol

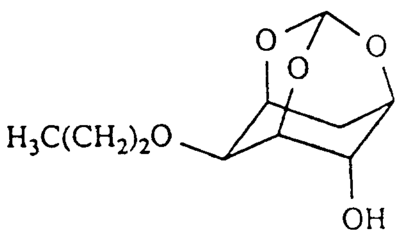

500 mg (1.64 mmol) of compound of Example 20 are dissolved in 10 ml of ethyl acetate and 500 mg of palladium hydroxide (10 %) on charcoal are added to the solution. The mixture is stirred for 1 h under hydrogen (27.6 × $10^3$ Pa (4 psi)). After filtering on Whatman paper, eluting with ethyl acetate and evaporating to dryness, 320 mg (1.55 mmol) of title compound are isolated which compound crystallises from an ethyl acetate/n-pentane mixture (yield: 94 %).
M.p.: 161-163 ° C.
$[\alpha]_D$ = +1.0 ° (c = 0.9, $CH_2Cl_2$).

Example 22

2-O-propyl-6-deoxy-D-myoinositol

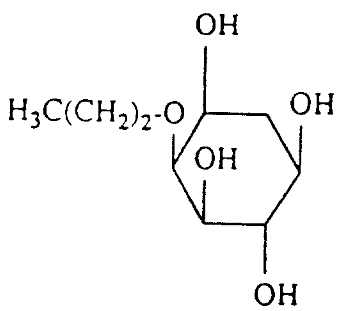

During the treatment of 300 mg (1.38 mmol) of compound of Example 21 with 10 ml of a 1N hydrochloric acid solution in a methanol/water mixture followed by evaporation to dryness, the title compound is obtained with a yield of 98 %. Purification takes place by crystallising from hexane.
M.p.: 138-140 ° C.
$[\alpha]_D$ = +6 ° (c = 1.3, $CH_3OH$).

Example 23

4-O-benzyl-1,3,5-O-orthoformyl-2-O-myristoyl-6-deoxy-D-myoinositol

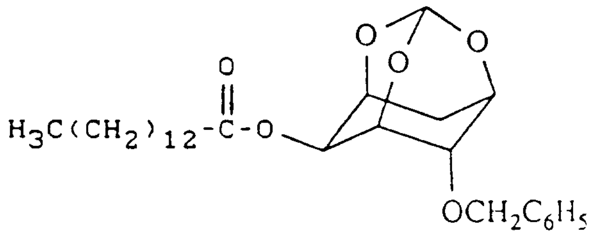

309 mg (1.5 mmol) of dicyclohexylcarbodiimide, 342 mg (1.5 mmol) of myristic acid and 20 mg of dimethylaminopyridine are added to 264 mg (1 mmol) of compound of Example 19, which is dissolved in 20 ml of anhydrous dichloromethane. At the end of stirring for 4 h at room temperature, the mixture is filtered under argon (elution: $CH_2Cl_2$). The filtrate is then evaporated to dryness and separated by flash chromatography on silica gel (hexane/ethyl acetate: 8/2). 450 mg (0.95 mmol) of title compound are isolated by crystallising from an ethyl acetate/pentane mixture (yield: 95 %).
M.p.: 35-37 °C.

Example 24

1,3,5-O-orthoformyl-2-O-myristoyl-6-deoxy-D-myoinositol

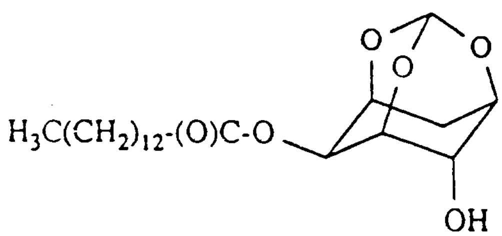

380 mg (0.8 mmol) of compound of Example 23, dissolved in 20 ml of ethanol, are hydrogenated for 4 h in a Parr apparatus in the presence of 400 mg of palladium-on-charcoal (10 %). After filtering on Whatman paper (elution: EtOH), the filtrate is evaporated to dryness. 307 mg (0.8 mmol) of title compound are isolated by crystallising from a methanol/water mixture (yield: 100 %).
M.p.: 99-101 °C.

Example 25

2-O-myristoyl-6-deoxy-D-myoinositol

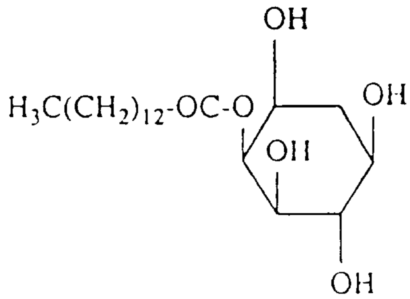

250 mg (0.65 mmol) of compound of Example 24 are treated for 4 h at 25 °C with a 1N solution of hydrochloric acid in methanol. After evaporating to dryness, purification of 243 mg (0.65 mmol) of the title compound takes place by crystallising from a methanol/water mixture (yield: 100 %).
M.p.: 142-144 °C.
$[\alpha]_D$ = -13 ° (c = 1, pyridine).

Example 26

4-O-benzyl-2,3-O-cyclohexylidene-1,6-dideoxymyoinositol 1-methylidene(diethyl phosphonate)

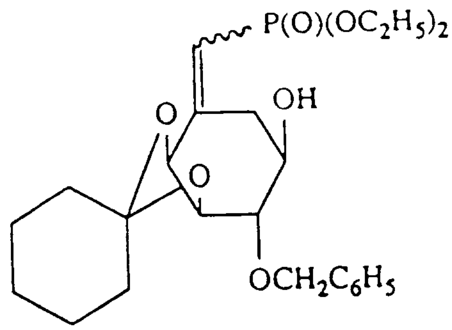

5 mg of bipyridyl and, dropwise, 2.9 ml of n-butyllithium (n-BuLi) (1.4N) are added at 0 °C to a solution of 1 ml (4 mmol) of tetraethyl methylenebisphosphonate in 10 ml of anhydrous tetrahydrofuran. The addition of n-BuLi is interrupted as soon as the solution becomes red in colour. At the end of stirring for 1/2 h, 664 mg (2 mmol) of compound of Example 6, dissolved in 10 ml of anhydrous THF, are added to the phosphonate anion formed.

After the reaction, ice and ammonium chloride are added. The solution is then neutralised with acetic acid and extracted with ethyl acetate. The organic phase is evaporated to dryness and the residue is separated by chromatography on a column of Florisil under moderate pressure (elution: ethyl acetate/hexane: 6/4). 1.63 mg of 1-methylidenephosphonate appears in the form of diastereoisomers in a 3.5/1.5 ratio. The overall yield of the reaction is greater than 70 %.

Example 27

2,3-O-cyclohexylidene-1,6-dideoxy-D-myoinositol 1-C-methylene(diethylphosphonate)-4,5-bis(dibenzylphosphate)

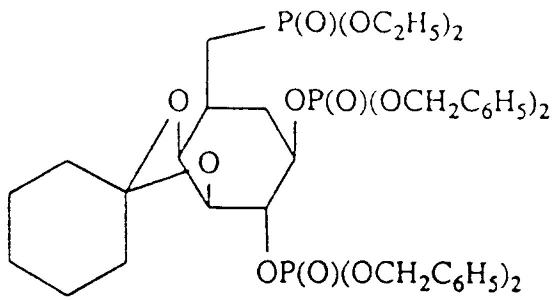

The preparation of the title compound takes place in two stages starting from the compound of Example 26.

a) Preparation of the intermediate product 2,3-O-cyclohexylidene-1,6-dideoxymyoinositol 1-C-methylene-(diethyl phosphonate) (Example 28)

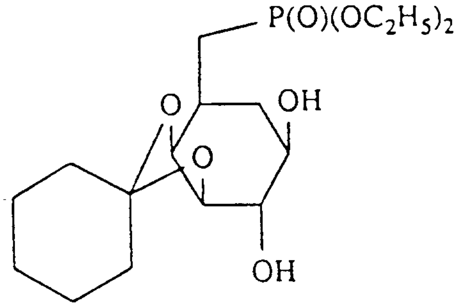

(Example 28)

500 mg (1.07 mmol) of the compound of Example 26, dissolved in an ethyl acetate/EtOH mixture, are hydrogenated in a Parr apparatus at a pressure of 34.5 × $10^3$ Pa (5 psi) in the presence of 500 mg of palladium-on-charcoal (5 %). At the end of stirring for 1.5 h, the mixture is filtered on Whatman paper and the filtrate is evaporated to dryness. The 380 mg (1 mmol) of crude product obtained are then phosphorylated.

b) Preparation of 27 from 28

380 mg (1 mmol) of compound of Example 28 are phosphorylated according to Phosphoramidite Process A. The title compound is isolated with a yield of 70 % (oily product).

$[\alpha]_D$ = +3.75° (c = 0.8, $CHCl_3$).

Example 29

6-deoxy-D-myoinositol 1,4,5-tris(dibenzyl phosphate)

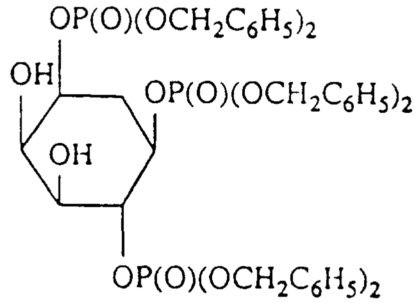

163 mg (0.15 mmol) of compound of Example 12 are treated for 2 h at room temperature with a 1N solution of hydrochloric acid in methanol. The solution is evaporated to dryness. 110 mg (0.115 mmol) of title compound appear after separating by flash chromatography an silica gel [eluent: 1) ethyl acetate/hexane: 8/2; 2) ethyl acetate; 3) ethyl acetate/MeOH: 9/1] and crystallise from an ethyl acetate/pentane mixture (yield: 76 %).
M.p.: 122-123 °C.
$[\alpha]_D$ = 0 °.

Preparation examples

Example I

3-O-myristoyl-6-deoxy-D-myoinositol 1,4,5-tris(dibenzyl phosphate)

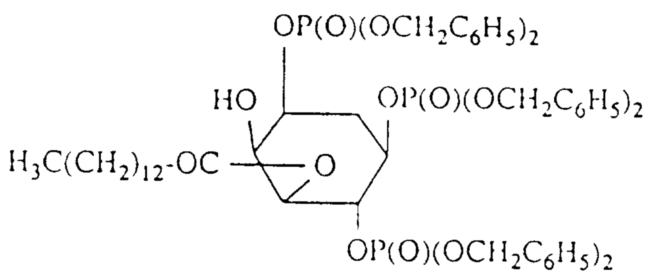

41 mg (0.12 mmol) of dicyclohexylcarbodiimide, 25 mg (0.12 mmol) of myristic acid and 20 mg of dimethylaminopyridine are added to 95 mg (0.1 mmol) of compound of Example 29, which is dissolved in 10 ml of anhydrous dichloromethane. After stirring for 4 h at room temperature, the mixture is filtered on diatomaceous earth (elution: $CH_2Cl_2$), evaporated to dryness and separated by flash chromatography on silica gel (eluent: ethyl acetate/heptane). The title compound is purified by crystallising from an ethyl acetate/Hp mixture (yield: 60 %).
M.p.: 88-90 °C.
$[\alpha]_D$ = 0 °.

Example II

2,3-di-O-myristoyl-6-deoxy-D-myoinositol 1,4,5-tris(dibenzyl phosphate)

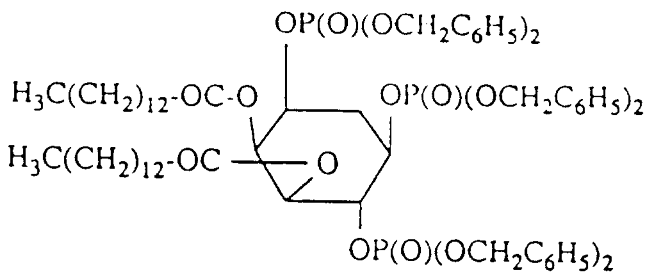

By analogy with the instructions in Example I, the title compound is prepared in the form of an oil from the compound of Example 29 using 0.24 mmol of myristic acid and 0.24 mmol of DCC with a yield of 84 %. $[\alpha]_D = 0°$.

Example III

3-O-myristoyl-6-deoxy-D-myoinositol 1,4,5-triphosphate

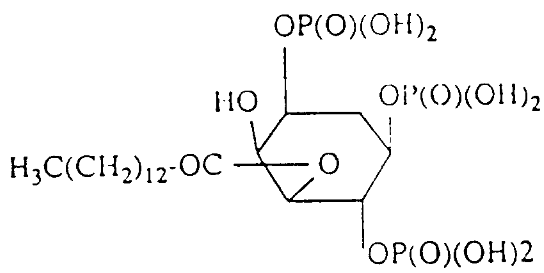

69 mg of compound of Example I are hydrogenated in ethanol at a pressure of $34.5 \times 10^3$ Pa (5 psi) in the presence of 100 mg of palladium-on-charcoal (10 %). After filtering on Whatman paper (elution with water) and partially evaporating the aqueous phase, 6 mol equivalents of Tris salt are added. After lyophilising, the title compound is isolated in the form of the solid hexatris salt.
$M^+H = 1358$

| Analysis : $C_{44}H_{107}O_{34}N_6P_3$ | | | | |
|---|---|---|---|---|
| calculated<br>found | C 38.93;<br>C 38.75; | H 7.95;<br>H 7.22; | N 6.1;<br>N 6.42; | P 6.85.<br>P 7.02. |

Example IV

2,3-di-O-myristoyl-6-deoxy-D-myoinositol 1,4,5-triphosphate

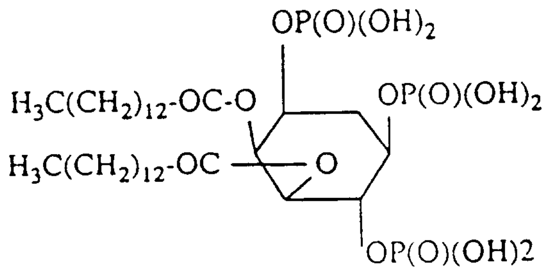

By analogy with the instructions of Example III, the title compound is obtained in the form of the hexatris salt from the compound of Example II.

| Analysis : $C_{59}H_{133}O_{36}N_6P_3$ | | | | |
|---|---|---|---|---|
| calculated<br>found | C 43.98;<br>C 44.01; | H 8.46;<br>H 8.52; | N 5.31;<br>N 5.27; | P 5.87.<br>P 5.0. |

Example V

2-O-benzyl-6-deoxy-D-myoinositol 1,3,4,5-tetrakis(dibenzyl phosphate)

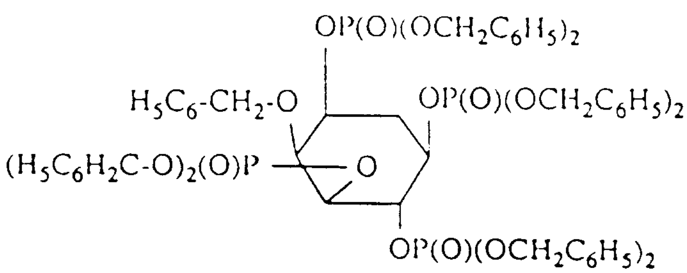

374 mg (1 mmol) of the compound of Example 16 are phosphorylated by Phosphoramidite Process A with a yield of 46 %. The title compound is isolated in the form of a yellow oil after separating on Florisil. $[\alpha]_D = 0°$.

Example VI

6-deoxy-D-myoinositol 1,3,4,5-tetrakis(dibenzyl phosphate)

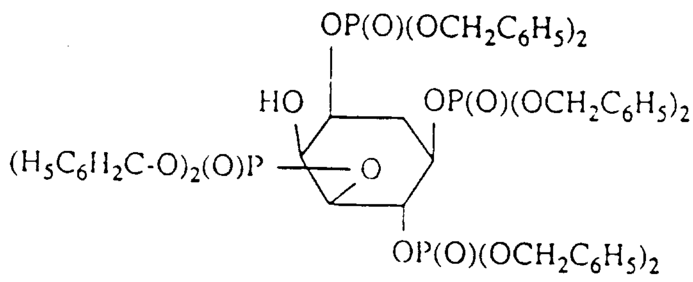

The compound of Example 29 is selectively phosphorylated to the title compound with a yield of 70 % (in the form of an oil) according to Pyrophosphate Process B and by using 1.2 equivalents of tetrabenzyl pyrophosphate.
$[\alpha]_D$ = +1° (0.98, $CHCl_3$).

Example VII

6-deoxy-D-myoinositol 1,3,4,5-tetraphosphate

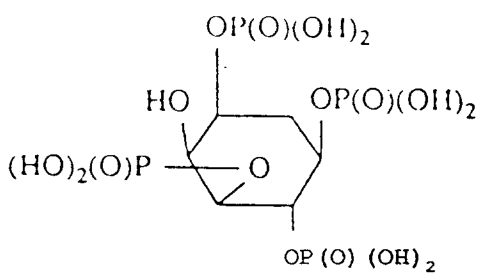

The compound of Example VI is treated according to Process C in order to obtain the title compound in the form of the octatris salt (8 equivalents of Tris) (in the form of an oil).
$[\alpha]_D$ = 0°.

| Analysis : $C_{38}H_{104}O_{41}N_8P_4 \cdot 4H_2O$. | | | |
|---|---|---|---|
| calculated<br>found | C 29.92;<br>C 30.21; | H 7.40;<br>H 7.35; | N 7.35.<br>N 7.52. |

Example VIII

2-O-propyl-6-deoxy-D-myoinositol 1,3,4,5-tetrakis(dibenzyl phosphate)

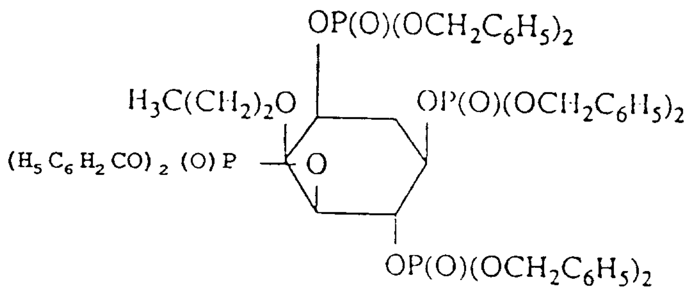

The compound of Example 22 is phosphorylated with a yield of 65 % according to Phosphoramidite Process A.
$[\alpha]_D$ = +3° (c = 0.77, $CH_2Cl_2$).

Example IX

2-O-propyl-6-deoxy-D-myoinositol 1,3,4,5-tetraphosphate

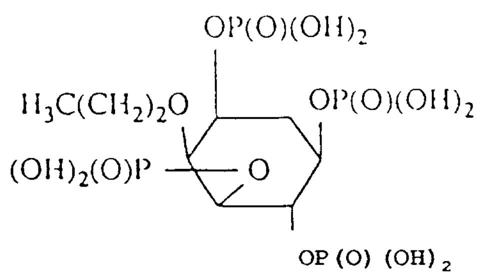

The title compound appears in the form of the octatris salt from the compound of Example V treated according to Process C.
$[\alpha]_D$ = 0.

Examples X and XI

2-O-myristoyl-6-deoxy-D-myoinositol 1,3,4,5-tetrakis(dibenzyl phosphate)

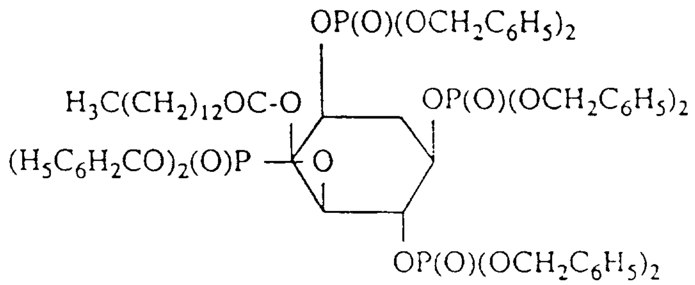

(Example X)

The title compound is obtained, by Phosphoramidite Process A, in the form of an oil from the compound of Example 25 with a yield of 40 %.

2-O-myristoyl-6-deoxy-D-myoinositol 1,3,4,5-tetraphosphate

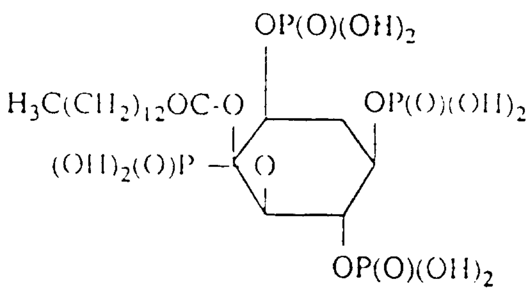

(Example XI)

142 mg (0.1 mmol) of compound of Example VII are hydrogenated at a pressure of 5 × $10^5$ Pa (5 bar) in an ethanol solution with 150 mg of 10 % palladium-on-charcoal. The solution is filtered, concentrated and 97 mg (8 equivalents) of Tris salt are twice added. After lyophilising, 165 mg of title compound are obtained.

Example XII

2-O-benzyl-3,6-dideoxy-D-myoinositol 1,4,5-tris(dibenzyl phosphate)

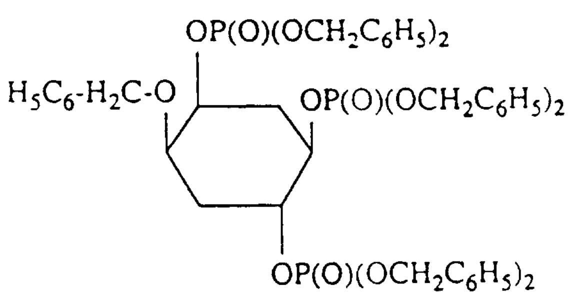

The title compound is obtained from 300 mg (1.26 mmol) of 2-O-benzyl-3-dideoxy-D-myoinositol, by Phosphoramidite Process A, with a yield of 20 % in the form of an oil.
$[\alpha]_D = 0°$.

Example XIII

3,6-dideoxy-D-myoinositol 1,4,5-triphosphate

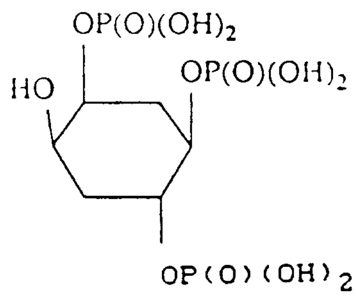

50 mg of palladium-on-charcoal (10 %) are added to 50 mg (0.049 mmol) of compound of Example XII, which are dissolved in 5 ml of ethanol. The solution is hydrogenated for 3 h at a pressure of $20.7 \times 10^3$ Pa (3 psi). After filtering on Whatman paper, 2 times 18 mg of Tris are added. The title compound is isolated by lyophilising.
$[\alpha]_D = 0°$.

Example XIV

6-deoxy-D-myoinositol 1-C-methylene(diethyl phosphonate)-4,5-diphosphate

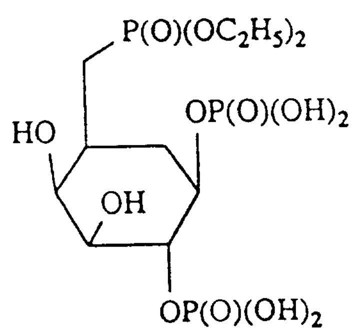

200 mg (0.22 mmol) of compound of Example 27, dissolved in 2 ml of ethanol, are hydrogenated for 1 h at a pressure of $34.5 \times 10^3$ Pa (5 psi) in the presence of 200 mg of palladium-on-charcoal (10 %). The mixture is then filtered on Whatman paper and eluted with distilled water. The solution is partially evaporated in a rotary evaporator and 4 equivalents (27 mg) of tris(hydroxymethyl)aminomethane (Tris) are added. After lyophilising, the title compound is in the form of the tetratris salt.

Example XV

6-deoxy-D-myoinositol 1-C-methylenephosphonate-4,5-diphosphate

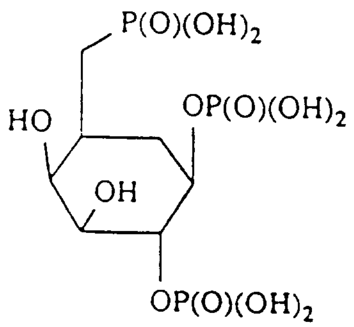

30 mg of pieces of sodium are added to 179 mg (0.2 mmol) of compound of Example 27, which are dissolved in 1 ml of condensed ammonia solution at -60 °C. After the end of the reaction, monitored by thin layer chromatography, the solution is heated again to room temperature and partially evaporated. The residue is diluted with distilled water and filtered on acidic resin IRC 50. After additions of 6 equivalents of Tris, the title compound is obtained after lyophilising.

Example XVI

3,6-Dideoxy-D-myoinositol-3,4,5-tri(dibutyl) phosphate

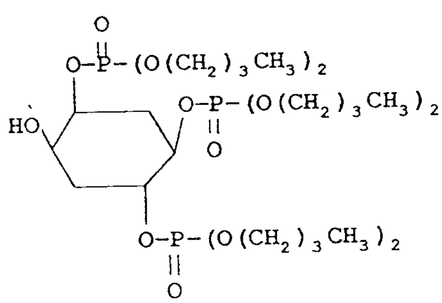

| Analysis: $C_{30}H_{63}O_{13}P_3$ | | | | |
|---|---|---|---|---|
| calculated:<br>found: | C 49,71;<br>C 48,91; | H 8,76;<br>H 8,48; | O 28,70; | P 12,82<br>P 12,67 |

## Claims

1. Deoxycyclitol derivatives, characterised in that they correspond to the general formula:

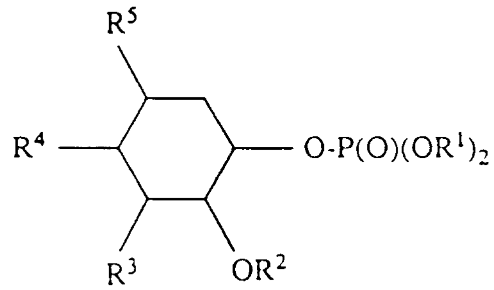

(I)

in which

| | |
|---|---|
| $R^1$ | represents a hydrogen atom, a linear or branched alkyl group having up to 8 carbon atoms or a protecting group of the hydroxyl groups, |
| $R^2$ | represents hydrogen or a residue of formula $-P(O)(OR^6)_2$, in which |
| $R^6$ | has the meaning shown above for $R^1$ and is identical to the latter or different from the latter, |
| $R^4$ | represents a residue of formula $-OP(O)(OR^7)_2$ or A-O-, in which |
| $R^7$ | also has the meaning shown above for $R^1$ and is identical to the latter or different from the latter, |
| A | represents a hydrogen atom or a linear or branched alkyl group having up to 10 carbon atoms or a linear or branched acyl group having up to 18 carbon atoms, |
| $R^3$ | has the meaning shown above for $R^4$ and is identical to the latter or different from the latter or represents a hydrogen atom, |
| $R^5$ | represents a group of formula $-OP(O)(OR^8)_2$ or $-CH_2P(O)(OR^9)_2$, in which |
| $R^8$ and $R^9$ | are identical or different and have the meaning shown above for $R^1$, |

and their salts, with the condition that, when 2 or 3 of the substituents shown above represent the protecting group of the hydroxyl groups, the remaining substituents must not represent the residues of phosphoric acid $-OP(O)(OH)_2$ or $-P(O)(OH)_2$,

and

$R^1$ must not represent hydrogen when $R^2$ represents the group of formula $-P(O)(OH)_2$ and $R^3$, $R^4$ and $R^5$ each represent the group of formula $-OP(O)(OH)_2$.

**2.** Derivatives according to Claim 1, characterised in that

| | |
|---|---|
| $R^1$ | represents a hydrogen atom, a linear or branched alkyl group having up to 6 carbon atoms or a benzyl, benzyloxycarbonyl or 2- or 4-nitrobenzyl group, |
| $R^2$ | represents a hydrogen atom, a benzyl group or a residue of formula $-P(O)(OR^6)_2$ in which |
| $R^6$ | has the meaning shown above for $R^1$ and is identical to the latter or is different therefrom, |
| $R^4$ | represents a residue of formula $-OP(O)(OR^7)_2$ or A-O-, in which |
| $R^7$ | also has the meaning given above for $R^1$ and is identical to the latter or is different therefrom, |
| A | represents a hydrogen atom, a benzyl group or a linear or branched alkyl group having up to 8 carbon atoms or a linear or branched acyl group having up to 16 carbon atoms, |
| $R^3$ | has the meaning shown above for $R^4$ and is identical to the latter or is different therefrom or represents a hydrogen atom, |
| $R^5$ | represents a group of formula $-OP(O)(OR^8)_2$ or $-CH_2P(O)(OR^9)_2$, in which |
| $R^8$ and $R^9$ | are identical or different and have the meaning shown above for $R^1$, |

and their salts, with the condition that, when 2 or 3 of the substituents shown above represent the protecting group of the hydroxyl groups, the remaining substituents must not represent the residues of phosphoric acid $-OP(O)(OH)_2$ or $-P(O)(OH)_2$,

and

$R^1$ must not represent a hydrogen atom when $R^2$ represents the group of formula $-P(O)(OH)_2$ and $R^3$, $R^4$ and $R^5$ each represent the group of formula $-OP(O)(OH)_2$.

**3.** Derivatives according to Claim 1, characterised in that

$R^1$ represents a hydrogen atom, a linear or branched alkyl group having up to 5 carbon atoms or a benzyl group,

$R^2$ represents a hydrogen atom, a benzyl group or a residue of formula $-P(O)(OR^6)_2$, in which

$R^6$ has the meaning shown above for $R^1$ and is identical to the latter or different therefrom,

$R^4$ represents a residue of formula $-OP(O)(OR^7)_2$ or A-O-, in which

$R^7$ also has the meaning shown above for $R^1$ and is identical to the latter or is different therefrom, and

A represents a hydrogen atom, a benzyl group or a linear or branched alkyl group having up to 6 carbon atoms or a linear or branched acyl group having up to 14 carbon atoms,

$R^3$ has the meaning shown above for $R^4$ and is identical to the latter or is different therefrom or represents a hydrogen atom,

$R^5$ represents a group of formula $-OP(O)(OR^8)_2$ or $-CH_2P(O)(OR^9)_2$, in which

$R^8$ and $R^9$ are identical or different and have the meaning shown above for $R^1$,

and their salts, with the condition that, when 2 or 3 of the substituents shown above represent the protecting group of the hydroxyl groups, the remaining substituents must not represent the residues of phosphoric acid $-OP(O)(OH)_2$ or $-P(O)(OH)_2$,

and

$R^1$ must not represent a hydrogen atom when $R^2$ represents the group of formula $-P(O)(OH)_2$ and $R^3$, $R^4$ and $R^5$ each represent the group of formula $-OP(O)(OH)_2$.

**4.** Derivatives according to Claim 1, characterised in that they are intended for controlling diseases.

**5.** Process for preparing deoxycyclitol derivatives of general formula:

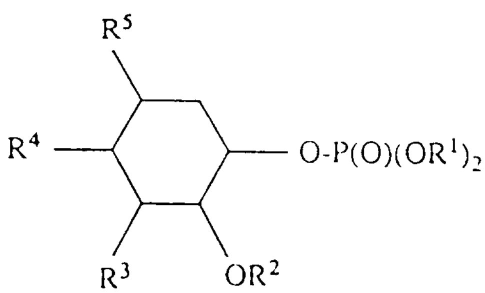

(I)

in which

$R^1$ represents a hydrogen atom, a linear or branched alkyl group having up to 8 carbon atoms or a protecting group of the hydroxyl groups,

$R^2$ represents hydrogen or a residue of formula $-P(O)(OR^6)_2$, in which

$R^6$ has the meaning shown above for $R^1$ and is identical to the latter or different from the latter,

$R^4$ represents a residue of formula $-OP(O)(OR^7)_2$ or A-O-, in which

$R^7$ also has the meaning shown above for $R^1$ and is identical to the latter or different from the latter,

A represents a hydrogen atom or a linear or branched alkyl group having up to 10 carbon atoms or a linear or branched acyl group having up to 18 carbon atoms,

$R^3$ has the meaning shown above for $R^4$ and is identical to the latter or different from the latter or represents a hydrogen atom,

$R^5$ represents a group of formula $-OP(O)(OR^8)_2$ or $-CH_2P(O)(OR^9)_2$,

in which

$R^8$ and $R^9$ are identical or different and have the meaning shown above for $R^1$,

and their salts, with the condition that, when 2 or 3 of the substituents shown above represent the protecting group of the hydroxyl groups, the remaining substituents must not represent the residues of phosphoric acid $-OP(O)(OH)_2$ or $-P(O)(OH)_2$,

and

$R^1$ must not represent hydrogen when $R^2$ represents the group of formula $-P(O)(OH)_2$ and $R^3$, $R^4$ and $R^5$ each represent the group of formula $-OP(O)(OH)_2$,

characterised in that

[A] compounds of general formula (II)

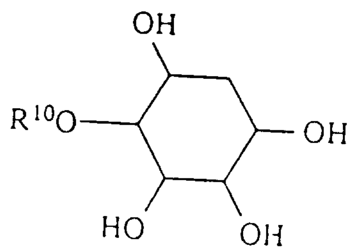

(II)

in which

$R^{10}$ represents one of the protecting groups of the hydroxyl groups mentioned above, preferably a benzyl group,

are reacted with compounds of general formula (III)

$((CH_3)_2CH)_2N\text{-}P(OR^{11})_2$ (III)

in which

$R^{11}$ has the meaning shown above for $R^{10}$ and is identical to the latter or is different therefrom,

in inert solvents, optionally in the presence of a base and then of an oxidising agent in order to form the compounds of general formula (IV)

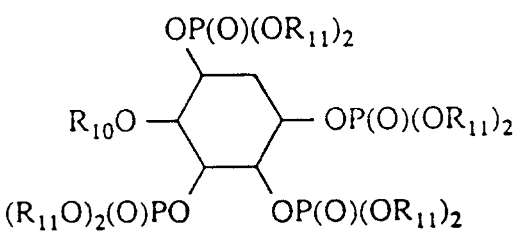

(IV)

in which

$R^{10}$ and $R^{11}$ have the meaning shown above,

or

compounds of general formula (V)

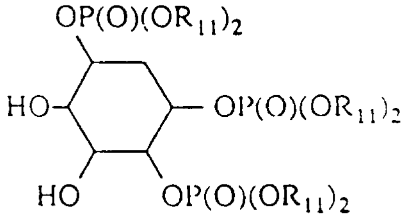

(V)

in which

$R^{11}$ has the meaning shown above,

are reacted with tetrabenzyl pyrophosphate, in inert solvents, in the presence of a base, in order to form the compounds of general formula (IVa)

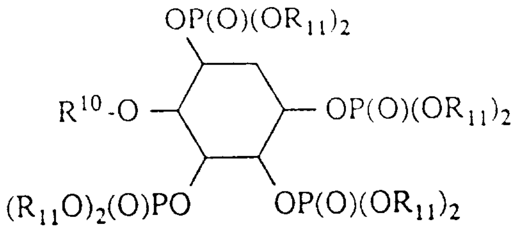

(IVa)

in which

$R^{10}$ and $R^{11}$ have the meaning shown above,

and then the protecting groups $R^{11}$ are removed by usual methods, preferably by catalytic hydrogenation, in the case of the compounds of general formula (IV) in the presence of tris-(hydroxymethyl)aminomethane,

or else

[B] when $R^3$ and/or $R^4$ represent the residue of formula A-O-, compounds of general formula (V) are first reacted with the compounds of general formula (VI)

A'-B (VI)

in which

A' has the meaning shown above for A, but does not represent a hydrogen atom, and

B represents a hydroxyl group or a typical leaving group such as tosylate, mesylate, chlorine, bromine or iodine, preferably chlorine or iodine,

in inert solvents, optionally in the presence of a base and/or of an additional reactant, in order to form the compounds of general formula (VII) or (VIIa)

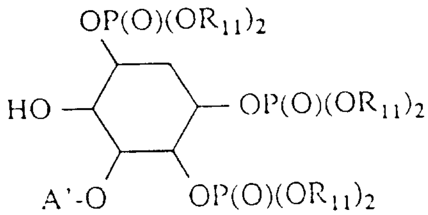

(VII)

or

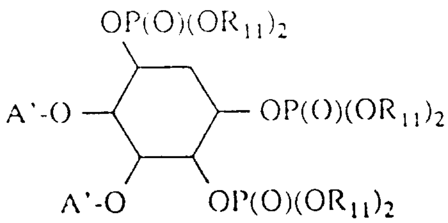

(VIIa)

in which

$R^{11}$ and A' have the meaning shown above,

or else

compounds of general formula (VIII)

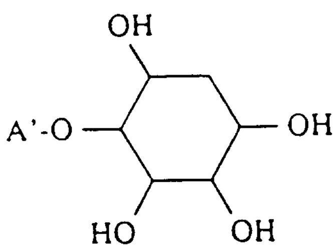

(VIII)

in which

A' has the meaning shown above,

are first of all converted to the compounds of general formula (IVb) by phosphorylation as described in [A],

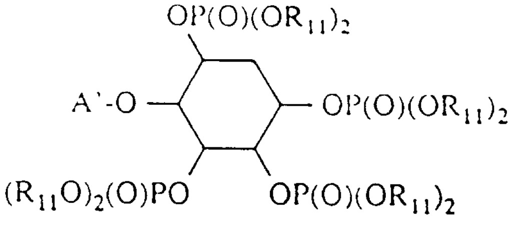

(IVb)

in which

A' and $R^{10}$ have the meaning shown above,

and, in a last stage, the various protecting groups of the compounds of general formulae (VII), (VIIa) and (IVb) are removed by the process described in [A],

or else

[C] when $R^3$ represents a hydrogen atom, compounds of general formula (IX)

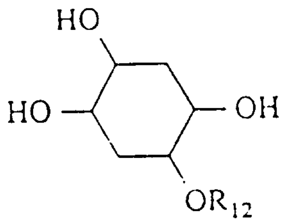

(IX)

in which

$R^{12}$ has the meaning shown above for $R^{10}$ and $R^{11}$ and is identical to these or is different therefrom,

are converted to the compounds of general formula (X) by phosphorylation according to the process described in [A]

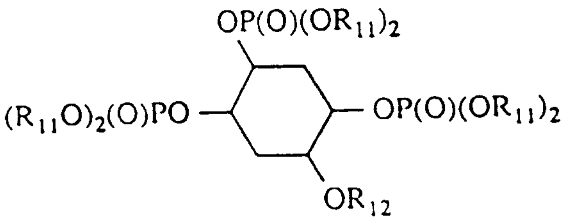

(X)

in which

$R^{11}$ and $R^{12}$ have the meaning shown above,

and then the protecting groups are removed by the process [A]

and

[D] when $R^5$ represents the residue of formula $-CH_2P(O)(OR^9)_2$, compounds of general formula (XI)

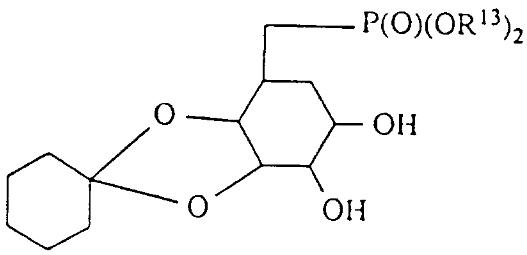

(XI)

in which

$R^{13}$ represents a $C_1$-$C_4$ alkyl group

are first reacted with the compounds of general formula (III) in order to form the compounds of general formula (XII)

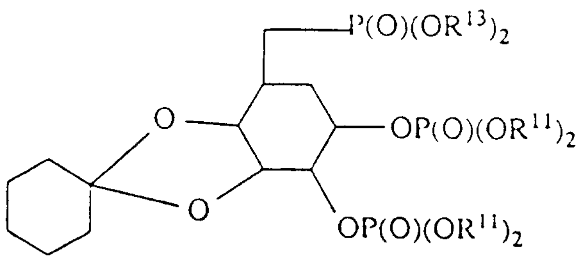

(XII)

in which $R^{11}$ and $R^{13}$ have the meaning shown above, and, in a later stage, the hydroxyl functional groups are freed with sodium in liquid ammonia with removal of all the protecting groups ($R^{11}$/$R^{13}$) or

by catalytic hydrogenation with the residues $R^{13}$ being retained.

**6.** Medicament, characterised in that it comprises at least one deoxycyclitol derivative according to Claim 1.

**7.** Medicament according to Claim 6, characterised in that it is intended for the treatment of inflammatory diseases.

**8.** Use of deoxycyclitol derivatives according to Claim 1 for treating diseases.

**9.** Use of deoxycyclitol derivatives according to Claim 1 for preparing medicaments.

**10.** Use of deoxycyclitol derivatives according to Claim 1 for preparing anti-inflammatory medicaments.